(19) Europäisches Patentamt European Patent Office Office européen des brevets

(11) **EP 4 408 284 B1**

(12) **EUROPEAN PATENT SPECIFICATION**

(45) Date of publication and mention
of the grant of the patent:
**27.05.2026 Bulletin 2026/22**

(21) Application number: **22793031.0**

(22) Date of filing: **27.09.2022**

(51) International Patent Classification (IPC):
***A61B 5/145*** (2006.01)

(52) Cooperative Patent Classification (CPC):
**A61B 5/14532;** A61B 2560/0252

(86) International application number:
**PCT/US2022/044891**

(87) International publication number:
**WO 2023/049509 (30.03.2023 Gazette 2023/13)**

(54) **ADAPTIVE SENSOR SENSITIVITY FOR ANALYTE MONITORING SYSTEMS**

ADAPTIVE SENSOREMPFINDLICHKEIT FÜR ANALYTÜBERWACHUNGSSYSTEME

SENSIBILITÉ DE CAPTEUR ADAPTATIVE POUR SYSTÈMES DE SURVEILLANCE D'ANALYTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2021 US 202163248786 P**

(43) Date of publication of application:
**07.08.2024 Bulletin 2024/32**

(73) Proprietor: **Abbott Diabetes Care Inc.
Alameda, CA 94502 (US)**

(72) Inventor: **COLE, Jean-Pierre J.
Tracy, CA 95304 (US)**

(74) Representative: **Mathys & Squire
32 London Bridge Street
The Shard
London SE1 9SG (GB)**

(56) References cited:
**US-A1- 2018 279 928    US-B2- 10 908 114
US-B2- 9 977 010**

## Description

FIELD

The disclosed subject matter relates to systems and methods for analyte monitoring, including adjusting sensitivity of an analyte sensor due to external factors.

BACKGROUND

The detection of the concentration level of glucose or other analytes in certain individuals using a medical sensor may be beneficial to their health. For example, the monitoring of glucose levels is important to individuals with diabetes or pre-diabetes. People with diabetes may need to monitor their glucose levels to determine when medication (e.g., insulin) is needed to reduce their glucose levels or when additional glucose is needed.

Devices and systems have been developed for automated in vivo monitoring of analyte concentrations, such as glucose levels, in bodily fluids such as in the blood stream or in interstitial fluid. Some of these analyte level measuring devices are configured so that at least a portion of the devices are positioned below a skin surface of a user, e.g., in a blood vessel or in the subcutaneous tissue of a user. As used herein, the term analyte monitoring system is used to refer to any type of in vivo monitoring system that uses a sensor disposed with at least a portion subcutaneously to measure and store sensor data representative of analyte concentration levels automatically over time. Analyte monitoring systems can include transmit sensor to a processor/display unit for further processing and/or display to a user.

Medical sensors used in analyte monitoring systems may lose sensitivity due to a change of temperature in an external environment of the sensors over time. Conventional sensor sensitivity correction systems using a fixed slope correction method may affect the sensor expiry, which can be based at least in part on a sensor sensitivity threshold. For example, in order to maintain a sensor sensitivity within a 15% change range, a sensor expiry period can be set to about 21 weeks. Taking the manufacturing margin into consideration, the sensor expiry can be further reduced to about 18 weeks. Thus, there is a need to reduce or eliminate the variation of sensor sensitivity over time to increase the lifespan of sensors and to adjust the sensor sensitivity.

Additionally, temperature monitoring during storage of medical sensors can be beneficial to quantify effects on sensor sensitivity and adaptively adjust sensor sensitivity accordingly. However, frequent temperature measurements can waste battery power and memory resources of medical sensors. Thus, there is a need to perform sensor sensitivity adjustment in an efficient and low-power manner. US 9977010 discloses end of life detection for analyte sensors and US 10908114 discloses compensation techniques for the effects of temperature on implantable sensors.

Accordingly, there is an opportunity for devices and systems that can be implemented by low-power, and low-cost, medical devices to efficiently adjust sensitivity of a medical sensor due to a change of temperature in an external environment of the sensor over time.

SUMMARY

The aspects and/or embodiments and/or examples disclosed in the following description, but that are not covered by the appended claims, are considered as not being part of the present invention and are disclosed by way of example only. The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the drawings.

To achieve these and other advantages and in accordance with the purpose of the disclosed subject matter, as embodied and broadly described, the disclosed subject matter includes analyte monitoring devices and computer program products stored on a computer-readable medium for monitoring an analyte.

According to one aspect of the disclosed subject matter, an analyte monitoring device according to the disclosed subject matter includes one or more processors, an analyte sensor, a temperature sensor, a communication module, and one or more memories communicatively coupled to the one or more processors, the analyte sensor, the temperature sensor, and the communication module. The processors are configured to: generate temperature data indicative of an external temperature of the analyte sensor measured by the temperature sensor corresponding to a first time, calculate a sensitivity adjustment of the analyte sensor based on the temperature data corresponding to the first time, add the sensitivity adjustment corresponding to the first time to a total sensitivity adjustment, and determine whether the first time is below a predetermined time threshold, and if the first time is below the predetermined time threshold, generate temperature data indicative of an external temperature of the analyte sensor measured by the temperature sensor corresponding to a second time and calculate an interval between the second time and the first time.

Additionally or alternatively, the processors can determine whether the first time is below a predetermined time threshold, and if the first time is equal to the predetermined time threshold, store the total sensitivity adjustment in one OTP memory. The processors can calculate the sensitivity adjustment of the analyte sensor based on the temperature data corresponding to the first time, by multiplying the external temperature corresponding to the first time by a time interval by a sensor sensitivity change rate M. The time interval can be a time interval between the second time and the first time.

Additionally or alternatively, the sensor sensitivity change rate can be calculated using

$$M = \frac{M_{Tmax}}{T_{max}}$$

, wherein $T_{max}$ is a predetermined maximum temperature for which a sensitivity adjustment is calculated, and wherein $M_{Tmax}$ is a sensor sensitivity change rate at the predetermined maximum temperature. As embodied herein, $T_{max}$ is 30 °C, wherein $M_{Tmax}$ is 0.003 % /hour, and wherein the time interval between the second time and the first time is 1 hour.

Additionally or alternatively, the processors can generate temperature data indicative of the temperature of the analyte sensor measured by the temperature sensor corresponding to a time when the temperature is equal to or below 0 °C, and calculate a sensitivity adjustment of the analyte sensor based on the temperature data corresponding to the time as 0. As embodied herein, the total sensitivity adjustment can be a sum of a plurality of sensitivity adjustments corresponding a plurality of times, and the plurality of times includes the first time and the second time.

Additionally or alternatively, the processors can determine whether the first time is below the predetermined time threshold, and if the first time exceeds the predetermined time threshold, calculate a fixed sensitivity adjustment, and to add the fixed sensitivity adjustment to the stored total sensitivity adjustment as a final sensitivity adjustment. As embodied herein, the fixed sensitivity adjustment is calculated by a maximum temperature for which a sensitivity adjustment is calculated, by a time interval, by a fixed sensor sensitivity change rate $M_f$. As embodied herein, the fixed sensor sensitivity change rate $M_f$ can be a sensor sensitivity change rate at a temperature of half of the predetermined maximum temperature.

Additionally or alternatively, the processors can perform a sensitivity adjustment to the analyte sensor, by dividing a sensor sensitivity of the analyte sensor by 100, and by multiplying by a difference between 100 and the final sensitivity adjustment.

According to another aspect of the disclosed subject matter, a corresponding computer program product stored on a computer-readable medium. The computer program product includes instructions to generate temperature data indicative of an external temperature of an analyte sensor operably connected to the computer-readable medium measured by a temperature sensor operably connected to the computer-readable medium corresponding to a first time, calculate a sensitivity adjustment of the analyte sensor based on the temperature data corresponding to the first time, add the sensitivity adjustment corresponding to the first time to a total sensitivity adjustment, and determine whether the first time is below a predetermined time threshold, and if the first time is below the predetermined time threshold, generate temperature data indicative of an external temperature of the analyte sensor measured by the temperature sensor corresponding to a second time and calculate an interval between the second time and the first time. Additionally or alternatively, the computer program product can include corresponding features of the processors according to the disclosed subject matter.

It is to be understood that both the foregoing general description and the following detailed description are exemplary and are intended to provide further explanation of the disclosed subject matter. The accompanying drawings, which are incorporated in and constitute part of this specification, are included to illustrate and provide a further understanding of the methods and systems of the disclosed subject matter. Together with the description, the drawings explain the principles of the disclosed subject matter.

BRIEF DESCRIPTION OF THE DRAWINGS

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.

FIG. 1A is a system overview of a sensor applicator, reader device, monitoring system, network, and remote system.

FIG. 1B is a diagram illustrating an operating environment of an example analyte monitoring system for use with the techniques described herein.

FIG. 2A is a block diagram depicting an example embodiment of a reader device.

FIG. 2B is a block diagram illustrating an example data receiving device for communicating with the sensor according to exemplary embodiments of the disclosed subject matter.

FIGS. 2C and 2D are block diagrams depicting example embodiments of sensor control devices.

FIG. 2E is a block diagram illustrating an example analyte sensor according to exemplary embodiments of the disclosed subject matter.

FIG. 3A is a proximal perspective view depicting an example embodiment of a user preparing a tray for an assembly.

FIG. 3B is a side view depicting an example embodiment of a user preparing an applicator device for an assembly.

FIG. 3C is a proximal perspective view depicting an example embodiment of a user inserting an applicator device into a tray during an assembly.

FIG. 3D is a proximal perspective view depicting an example embodiment of a user removing an applicator device from a tray during an assembly.

FIG. 3E is a proximal perspective view depicting an example embodiment of a patient applying a sensor using an applicator device.

FIG. 3F is a proximal perspective view depicting an example embodiment of a patient with an applied sensor and a used applicator device.

FIG. 4A is a side view depicting an example embodiment of an applicator device coupled with a cap.

FIG. 4B is a side perspective view depicting an example embodiment of an applicator device and cap decoupled.

FIG. 4C is a perspective view depicting an example embodiment of a distal end of an applicator device and electronics housing.

FIG. 4D is a top perspective view of an exemplary applicator device in accordance with the disclosed subject matter.

FIG. 4E is a bottom perspective view of the applicator device of FIG. 4D.

FIG. 4F is an exploded view of the applicator device of FIG. 4D.

FIG. 4G is a side cutaway view of the applicator device of FIG. 4D.

FIG. 5 is a proximal perspective view depicting an example embodiment of a tray with sterilization lid coupled.

FIG. 6A is a proximal perspective cutaway view depicting an example embodiment of a tray with sensor delivery components.

FIG. 6B is a proximal perspective view depicting sensor delivery components.

FIGS. 7A and 7B are isometric exploded top and bottom views, respectively, of an exemplary sensor control device.

FIG. 8A-8C are assembly and cross-sectional views of an on-body device including an integrated connector for the sensor assembly.

FIGS. 9A and 9B are side and cross-sectional side views, respectively, of an example embodiment of the sensor applicator of FIG. 1A with the cap of FIG. 2C coupled thereto.

FIGS. 10A and 10B are isometric and side views, respectively, of another example sensor control device.

FIGS. 11A-11C are progressive cross-sectional side views showing assembly of the sensor applicator with the sensor control device of FIGS. 10A-10B.

FIGS. 12A-12C are progressive cross-sectional side views showing assembly and disassembly of an example embodiment of the sensor applicator with the sensor control device of FIGS. 10A-10B.

FIGS. 13A-13F illustrate cross-sectional views depicting an example embodiment of an applicator during a stage of deployment.

FIG. 14 is a graph depicting an example of an in vitro sensitivity of an analyte sensor.

FIG. 15 is a diagram illustrating example operational states of the sensor according to exemplary embodiments of the disclosed subject matter.

FIG. 16 is a diagram illustrating an example operational and data flow for over-the-air programming of a sensor according to the disclosed subject matter.

FIG. 17 is a diagram illustrating an example data flow for secure exchange of data between two devices according to the disclosed subject matter.

FIG. 18 is a diagram illustrating an example of adjusting sensor sensitivity using a fixed compensation during storage of the sensor and the changes of sensor sensitivity over time at different temperatures.

FIG. 19 is a flowchart illustrating an exemplary process to adjust the sensor sensitivity based on temperature data according to the invention.

FIG. 20 is a diagram illustrating the temperature changes, the sensitivity changes over time, and the sensitivity adjustment to the sensor according to the disclosed subject matter.

FIG. 21 is a diagram illustrating upper and lower error bounds for the sensitivity adjustment according to the disclosed subject matter.

## DETAILED DESCRIPTION

Reference will now be made in detail to the various exemplary embodiments of the disclosed subject matter, exemplary embodiments of which are illustrated in the accompanying drawings.

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for the use of analyte sensor insertion applicators for use with *in vivo* analyte monitoring systems. An applicator can be provided to the user in a sterile package with an electronics housing of the sensor control device contained therein. According to some embodiments, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other embodiments, the applicator, sensor control device, sensor module, and sharp module can be provided in a single package. The applicator can be used to position the sensor control device on a human body with a sensor in contact with the wearer's bodily fluid. The embodiments provided herein are improvements to reduce the likelihood that a sensor is improperly inserted or damaged, or elicits an adverse physiological response. Other improvements and advantages are provided as well. The various configurations of these devices are described in detail by way of the embodiments which are only examples.

Furthermore, many embodiments include *in vivo* analyte sensors structurally configured so that at least a portion of the sensor is, or can be, positioned in the body of a user to obtain information about at least one analyte of the body. It should be noted, however, that the embodiments disclosed herein can be used with *in vivo* analyte monitoring systems that incorporate *in vitro* capability, as well as purely *in vitro* or *ex vivo* analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps. These sensor control device embodiments can be used and can be capable of use to implement those steps performed by a sensor control device from any and all of the methods described herein.

Furthermore, the systems and methods presented herein can be used for operations of a sensor used in an analyte monitoring system, such as but not limited to wellness, fitness, dietary, research, information or any purposes involving analyte sensing over time. As used herein, "sensor" can refer to any device capable of receiving sensor information from a user, including for purpose of illustration but not limited to, body temperature sensors, blood pressure sensors, pulse or heart-rate sensors, glucose level sensors, analyte sensors, physical activity sensors, body movement sensors, or any other sensors for collecting physical or biological information. Analytes measured by the analyte sensors can include, by way of example and not limitation, glucose, ketones, lactate, oxygen, hemoglobin A1C, albumin, alcohol, alkaline phosphatase, alanine transaminase, aspartate aminotransferase, bilirubin, blood urea nitrogen, calcium, carbon dioxide, chloride, creatinine, hematocrit, lactate, magnesium, oxygen, pH, phosphorus, potassium, sodium, total protein, uric acid, etc.

Before describing these aspects of the embodiments in detail, however, it is first desirable to describe examples of devices that can be present within, for example, an *in vivo* analyte monitoring system, as well as examples of their operation, all of which can be used with the embodiments described herein.

There are various types of *in vivo* analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. *In vivo* analyte monitoring systems can also operate without the need for finger stick calibration.

*In vivo* analyte monitoring systems can be differentiated from *"in vitro"* systems that contact a biological sample outside of the body (or *"ex vivo"*) and that typically include a meter device that has a port for receiving an analyte test strip carrying bodily fluid of the user, which can be analyzed to determine the user's blood sugar level.

*In vivo* monitoring systems can include a sensor that, while positioned *in vivo,* makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

*In vivo* monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into *in vivo* and *in vitro* monitoring systems.

According to one aspect of the disclosed subject matter, an analyte monitoring device according to the disclosed subject matter includes one or more processors, an analyte sensor, a temperature sensor, a communication module, and one or more memories communicatively coupled to the one or more processors, the analyte sensor, the temperature sensor, and the communication module. The processors are configured to: generate temperature data indicative of an external temperature of the analyte sensor measured by the temperature sensor corresponding to a first time, calculate a sensitivity adjustment of the analyte sensor based on the temperature data corresponding to the first time, add the sensitivity adjustment corresponding to the first time to a total sensitivity adjustment, and determine whether the first time is below a predetermined time threshold, and if the first time is below the predetermined time threshold, generate temperature data indicative of an external temperature of the analyte sensor measured by the temperature sensor corresponding to a second time and calculate an interval between the second time and the first time.

Additionally or alternatively, the processors can determine whether the first time is below a predetermined time threshold, and if the first time is equal to the predetermined time threshold, store the total sensitivity adjustment in one one-time programmable memory. The processors can calculate the sensitivity adjustment of the analyte sensor based on the temperature data corresponding to the first time, by multiplying the external temperature corresponding to the first time by a time interval by a sensor sensitivity change rate. The time interval can be a time interval between the second time and the first time.

Additionally or alternatively, the sensor sensitivity change rate can be calculated using

$$M = \frac{M_{Tmax}}{T_{max}}$$ , wherein $T_{max}$ is a predetermined maximum temperature for which a sensitivity adjustment is calculated, and wherein $M_{Tmax}$ is a sensor sensitivity change rate at the predetermined maximum temperature. As embodied herein, $T_{max}$ is 30 °C, wherein $M_{Tmax}$ is 0.003 % /hour, and wherein the time interval between the second time and the first time is 1 hour.

Additionally or alternatively, the processors can generate temperature data indicative of the temperature of the analyte sensor measured by the temperature sensor corresponding to a time when the temperature is equal to or below 0 °C, and calculate a sensitivity adjustment of the analyte sensor based on the temperature data corresponding to the time as 0. As embodied herein, the total sensitivity adjustment can be a sum of a plurality of sensitivity adjustments corresponding a plurality of times, and the plurality of times includes the first time and the second time.

Additionally or alternatively, the processors can determine whether the first time is below the predetermined time threshold, and if the first time exceeds the predetermined time threshold, calculate a fixed sensitivity adjustment, and to add the fixed sensitivity adjustment to the stored total sensitivity adjustment as a final sensitivity adjustment. As embodied herein, the fixed sensitivity adjustment is calculated by a maximum temperature for which a sensitivity adjustment is calculated, by a time interval, by a fixed sensor sensitivity change rate. As embodied herein, the fixed sensor sensitivity change rate can be a sensor sensitivity change rate at a temperature of half of the predetermined maximum temperature.

Additionally or alternatively, the processors can perform a sensitivity adjustment to the analyte sensor, by dividing a sensor sensitivity of the analyte sensor by 100, and by multiplying by a difference between 100 and the final sensitivity adjustment.

According to another aspect of the disclosed subject matter, a corresponding computer program product stored on a computer-readable medium. The computer program product includes instructions to generate temperature data indicative of an external temperature of an analyte sensor operably connected to the computer-readable medium measured by a temperature sensor operably connected to the computer-readable medium corresponding to a first time, calculate a sensitivity adjustment of the analyte sensor based on the temperature data corresponding to the first time, add the sensitivity adjustment corresponding to the first time to a total sensitivity adjustment, and determine whether the first time is below a predetermined time threshold, and if the first time is below the predetermined time threshold, generate temperature data indicative of an external temperature of the analyte sensor measured by the temperature sensor corresponding to a second time and calculate an interval between the second time and the first time. Additionally or alternatively, the computer program product can include corresponding features of the processors according to the disclosed subject matter.

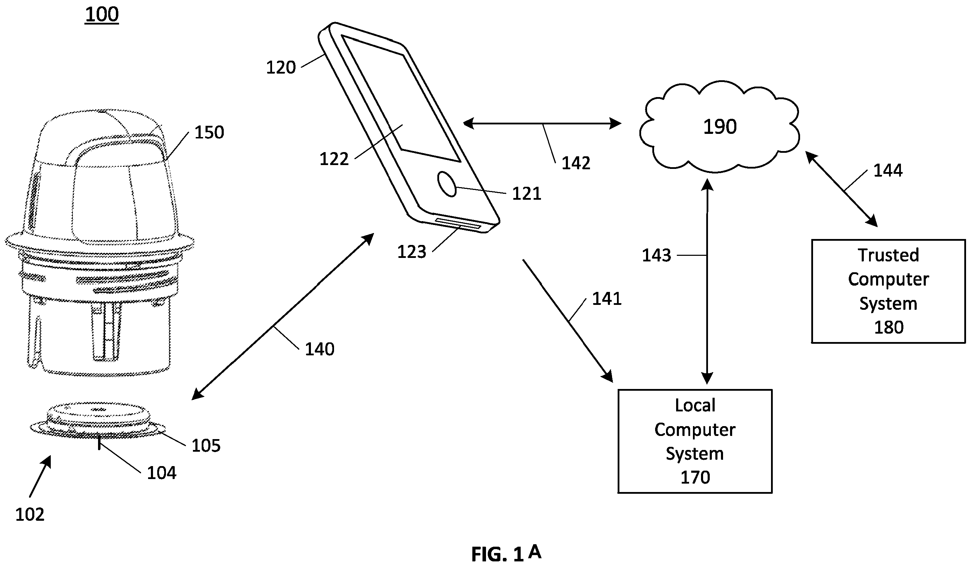

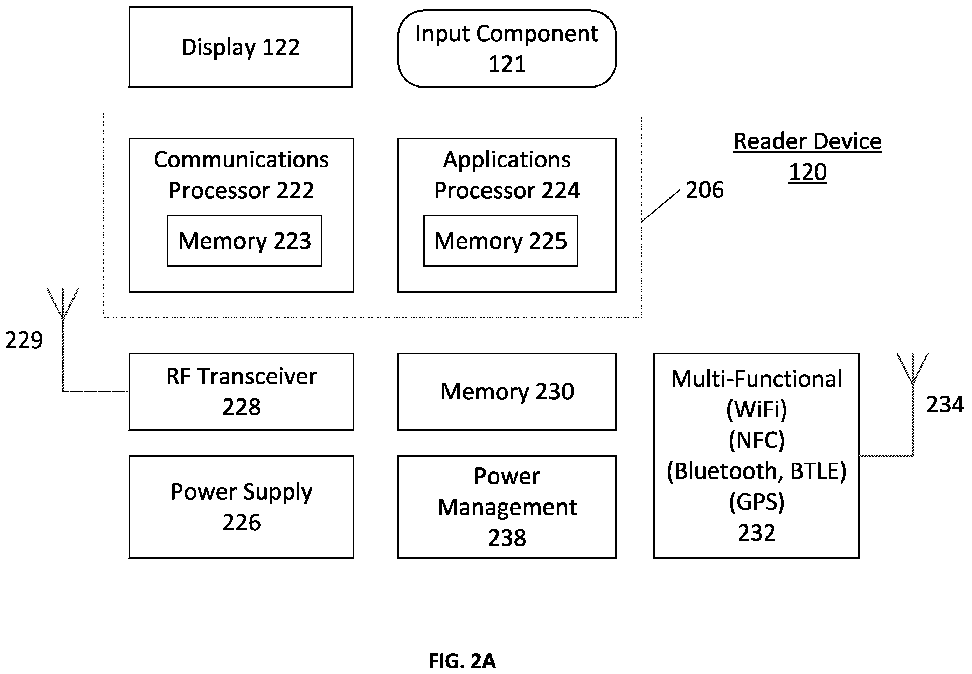

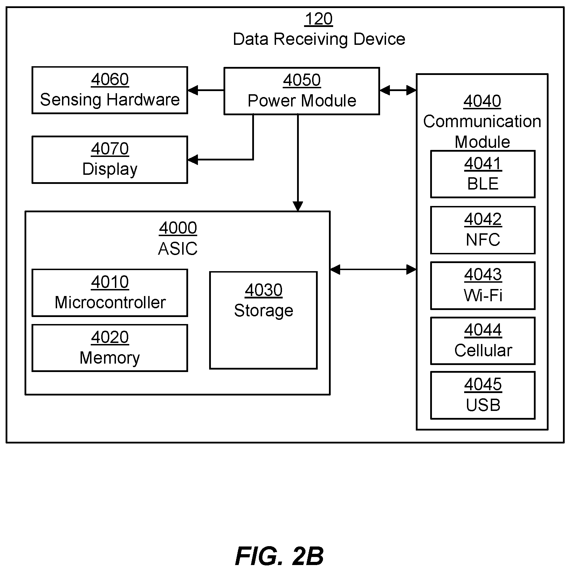

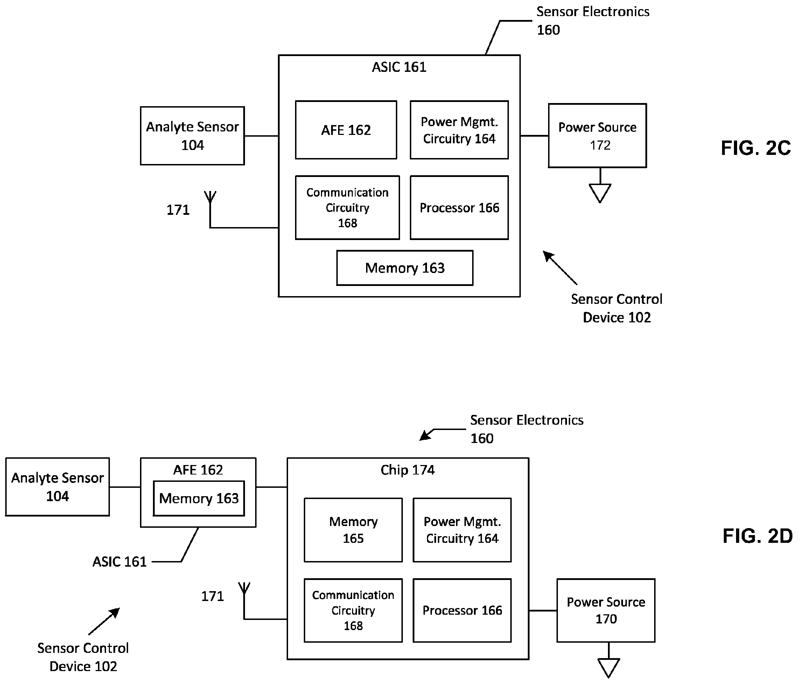

FIG. 1A is a conceptual diagram depicting an example embodiment of an analyte monitoring system 100 that includes a sensor applicator 150, a sensor control device 102, and a data receiving device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with data receiving device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on data receiving device 120 using screen 122 and input 121 and the device battery can be recharged using power port 123. More detail about data receiving device 120 is set forth with respect to FIG. 2A below. Data receiving device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how data receiving device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

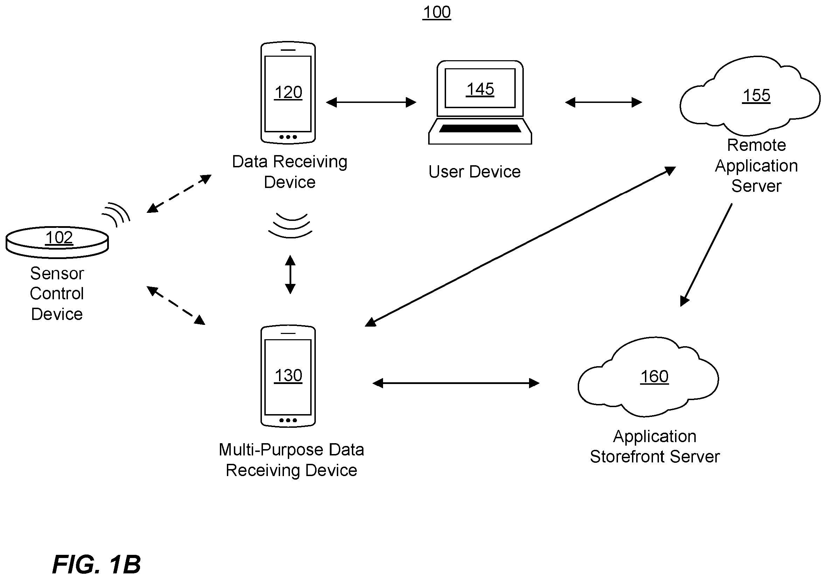

FIG. 1B illustrates another example embodiment of an operating environment of an analyte monitoring system 100 capable of embodying the techniques described herein. As illustrated, the analyte monitoring system 100 can include a system of components designed to provide monitoring of parameters, such as analyte levels, of a human or animal body or can provide for other operations based on the configurations of the various components. As embodied herein, the system can include a low-power sensor control device 102 worn by the user or attached to the body for which information is being collected. As embodied herein, the sensor control device 102 can be a sealed, disposable device with a predetermined active use lifetime (e.g., 1 day, 14 days, 30 days, etc.). Sensors 110 can be applied to the skin of the user body and remain adhered over the duration of the sensor lifetime or can be designed to be selectively removed and remain functional when reapplied. The low-power analyte monitoring system 100 can further include a data reading device 120 or multi-purpose data receiving device 130 configured as described herein to facilitate retrieval and delivery of data, including analyte data, from the sensor control device 102.

As embodied herein, the analyte monitoring system 100 can include a software or firmware library or application provided, for example via a remote application server 155 or application storefront server 160, to a third-party and incorporated into a multi-purpose hardware device 130 such as a mobile phone, tablet, personal computing device, or other similar computing device capable of communicating with the sensor control device 102 over a communication link. Multi-purpose hardware can further include embedded devices, including, but not limited to insulin pumps or insulin pens, having an embedded library configured to communicate with the sensor control device 102. Although the illustrated embodiments of the analyte monitoring system 100 include only one of each of the illustrated devices, this disclosure contemplates the analyte monitoring system 100 incorporate multiples of each components interacting throughout the system. For example and without limitation, as embodied herein, data receiving device 120 and/or multi-purpose data receiving device 130 can include multiples of each. As embodied herein, multiple data receiving devices 130 can communicate directly with sensor control device 102 as described herein. Additionally or alternatively, a data receiving device 130 can communicate with secondary data receiving devices 130 to provide analyte data, or visualization or analysis of the data, for secondary display to the user or other authorized parties.

FIG. 2A is a block diagram depicting an example embodiment of a data receiving device 120 configured as a smartphone. Here, data receiving device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

Data receiving device 120 can be a mobile communication device such as, for example, a Wi-Fi or internet enabled smartphone, tablet, or personal digital assistant (PDA). Examples of smartphones can include, but are not limited to, those phones based on a WINDOWS operating system, ANDROID operating system, IPHONE operating system, PALM, WEBOS, BLACKBERRY operating system, or SYMBIAN operating system, with data network connectivity functionality for data communication over an internet connection and/or a local area network (LAN).

Data receiving device 120 can also be configured as a mobile smart wearable electronics assembly, such as an optical assembly that is worn over or adjacent to the user's eye (e.g., a smart glass or smart glasses, such as GOOGLE GLASSES). This optical assembly can have a transparent display that displays information about the user's analyte level (as described herein) to the user while at the same time allowing the user to see through the display such that the user's overall vision is minimally obstructed. The optical assembly can be capable of wireless communications similar to a smartphone. Other examples of wearable electronics include devices that are worn around or in the proximity of the user's wrist (e.g., a smart watch, etc.), neck (e.g., a necklace, etc.), head (e.g., a headband, hat, etc.), chest, or the like.

For purpose of illustration and not limitation, reference is made to another exemplary embodiment of a data receiving device 120 for use with the disclosed subject matter as shown in FIG. 2B. The data receiving device 120, and the related multi-purpose data receiving device 130, includes components germane to the discussion of the sensor control device 102 and its operations and additional components can be included. In particular embodiments, the data receiving device 120 and multi-purpose data receiving device 130 can be or include components provided by a third party and are not necessarily restricted to include devices made by the same manufacturer as the sensor control device 102.

As illustrated in FIG. 2B, the data receiving device 120 includes an ASIC 4000 including a microcontroller 4010, memory 4020, and storage 4030 and communicatively coupled with a communication module 4040. Power for the components of the data receiving device 120 can be delivered by a power module 4050, which as embodied herein can include a rechargeable battery. The data receiving device 120 can further include a display 4070 for facilitating review of analyte data received from a sensor control device 102 or other device (e.g., user device 145 or remote application server 155). The data receiving device 120 can include separate user interface components (e.g., physical keys, light sensors, microphones, etc.).

The communication module 4040 can include a BLE module 4041 and an NFC module 4042. The data receiving device 120 can be configured to wirelessly couple with the sensor control device 102 and transmit commands to and receive data from the sensor control device 102. As embodied herein, the data receiving device 120 can be configured to operate, with respect to the sensor control device 102 as described herein, as an NFC scanner and a BLE end point via specific modules (e.g., BLE module 4042 or NFC module 4043) of the communication module 4040. For example, the data receiving device 120 can issue commands (e.g., activation commands for a data broadcast mode of the sensor; pairing commands to identify the data receiving device 120) to the sensor control device 102 using a first module of the communication module 4040 and receive data from and transmit data to the sensor control device 102 using a second module of the communication module 4040. The data receiving device 120 can be configured for communication with a user device 145 via a Universal Serial Bus (USB) module 4045 of the communication module 4040.

As another example, the communication module 4040 can include, for example, a cellular radio module 4044. The cellular radio module 4044 can include one or more radio transceivers for communicating using broadband cellular networks, including, but not limited to third generation (3G), fourth generation (4G), and fifth generation (5G) networks. Additionally, the communication module 4040 of the data receiving device 120 can include a Wi-Fi radio module 4043 for communication using a wireless local area network according to one or more of the IEEE 802.11 standards (e.g., 802.11a, 802.11b, 802.11g, 802.11n (aka Wi-Fi 4), 802.11ac (aka Wi-Fi 5), 802.11ax (aka Wi-Fi 6)). Using the cellular radio module 4044 or Wi-Fi radio module 4043, the data receiving device 120 can communicate with the remote application server 155 to receive analyte data or provide updates or input received from a user (e.g., through one or more user interfaces). Although not illustrated, the communication module 5040 of the analyte sensor 120 can similarly include a cellular radio module or Wi-Fi radio module.

As embodied herein, the on-board storage 4030 of the data receiving device 120 can store analyte data received from the sensor control device 102. Further, the data receiving device 120, multi-purpose data receiving device 130, or a user device 145 can be configured to communicate with a remote application server 155 via a wide area network. As embodied herein, the sensor control device 102 can provide data to the data receiving device 120 or multi-purpose data receiving device 130. The data receiving device 120 can transmit the data to the user computing device 145. The user computing device 145 (or the multi-purpose data receiving device 130) can in turn transmit that data to a remote application server 155 for processing and analysis.

As embodied herein, the data receiving device 120 can further include sensing hardware 4060 similar to, or expanded from, the sensing hardware 5060 of the sensor control device 102. In particular embodiments, the data receiving device 120 can be configured to operate in coordination with the sensor control device 102 and based on analyte data received from the sensor control device 102. As an example, where the sensor control device 102 glucose sensor, the data receiving device 120 can be or include an insulin pump or insulin injection pen. In coordination, the compatible device 130 can adjust an insulin dosage for a user based on glucose values received from the analyte sensor.

FIGS. 2C and 2D are block diagrams depicting example embodiments of sensor control device 102 having analyte sensor 104 and sensor electronics 160 (including analyte monitoring circuitry) that can have the majority of the processing capability for rendering end-result data suitable for display to the user. In FIG. 2C, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). **In** this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. **In** this embodiment, ASIC 161 is coupled with power source 172, which can be a coin cell battery, or the like. AFE 162 interfaces with *in vivo* analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to data receiving device 120 (not shown), for example, where minimal further processing is needed by the resident software application to display the data.

FIG. 2D is similar to FIG. 2C but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

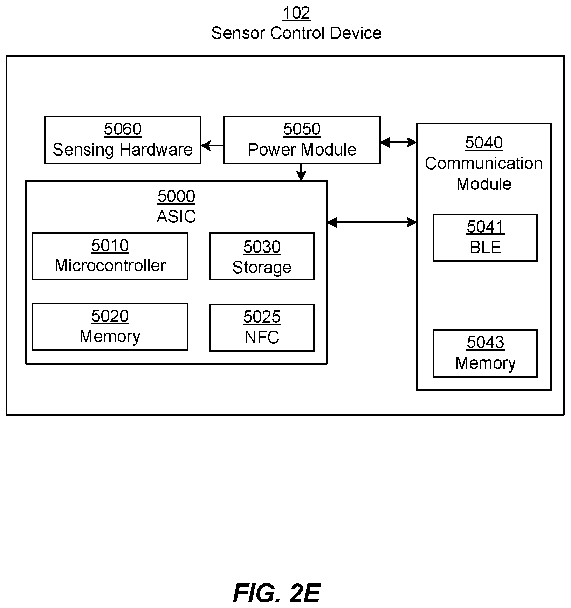

For purpose of illustration and not limitation, FIG. 2E depicts another exemplary embodiment of a sensor control device 102 compatible with the security architecture and communication schemes described herein.

As embodied herein, the sensor control device 102 can include an Application-Specific Integrated Circuit ("ASIC") 5000 communicatively coupled with a communication module 5040. The ASIC 5000 can include a microcontroller core 5010, on-board memory 5020, and storage memory 5030. The storage memory 5030 can store data used in an authentication and encryption security architecture. The storage memory 5030 can store programming instructions for sensor control device 102. As embodied herein, certain communication chipsets can be embedded in the ASIC 5000 (e.g., an NFC transceiver 5025). The ASIC 5000 can receive power from a power module 5050, such as an on-board battery or from an NFC pulse. The storage memory 5030 of the ASIC 5000 can be programmed to include information such as an identifier for sensor control device 102 for identification and tracking purposes. The storage memory 5030 can also be programmed with configuration or calibration parameters for use by sensor control device 102 and its various components. The storage memory 5030 can include rewritable or one-time programming (OTP) memory. The storage memory 5030 can be updated using techniques described herein to extend the usefulness of sensor control device 102.

As embodied herein, the communication module 5040 of sensor control device 102 can be or include one or more modules to support communications with other devices of an analyte monitoring system 100. As an example only, and not by way of limitation, example communication modules 5040 can include a Bluetooth Low-Energy ("BLE") module 5041 As used throughout this disclosure, BLE refers to a short-range communication protocol optimized to make pairing of Bluetooth devices simple for end users. The communication module 5040 can transmit and receive data and commands via interaction with similarly-capable communication modules of a data receiving device 120 or user device 145. The communication module 5040 can include additional or alternative chipsets for use with similar short-range communication schemes, such as a personal area network according to IEEE 802.15 protocols, IEEE 802.11 protocols, infrared communications according to the Infrared Data Association standards (IrDA), etc.

To perform its functionalities, the sensor control device 102 can further include suitable sensing hardware 5060 appropriate to its function. As embodied herein, the sensing hardware 5060 can include an analyte sensor transcutaneously or subcutaneously positioned in contact with a bodily fluid of a subject. The analyte sensor can generate sensor data containing values corresponding to levels of one or more analytes within the bodily fluid.

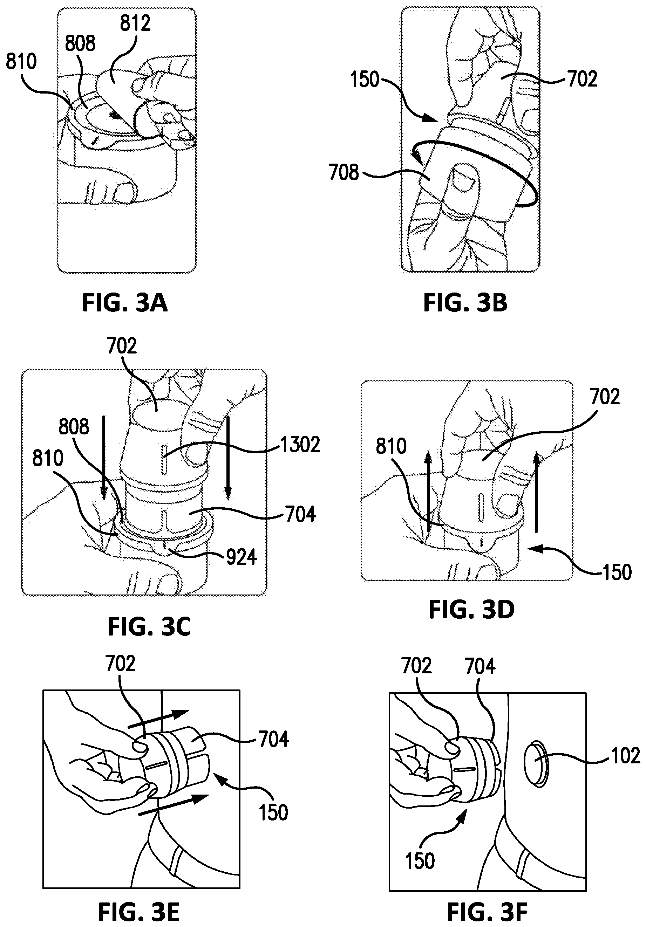

The components of sensor control device 102 can be acquired by a user in multiple packages requiring final assembly by the user before delivery to an appropriate user location. FIGS. 3A-3D depict an example embodiment of an assembly process for sensor control device 102 by a user, including preparation of separate components before coupling the components in order to ready the sensor for delivery. FIGS. 3E-3F depict an example embodiment of delivery of sensor control device 102 to an appropriate user location by selecting the appropriate delivery location and applying device 102 to the location.

FIG. 3A is a proximal perspective view depicting an example embodiment of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various embodiments so long as platform 808 is adequately exposed within tray 810. Lid 812 can then be placed aside.

FIG. 3B is a side view depicting an example embodiment of a user preparing an applicator device 150 for assembly. Applicator device 150 can be provided in a sterile package sealed by an applicator cap 708. Preparation of applicator device 150 can include uncoupling housing 702 from applicator cap 708 to expose sheath 704 (FIG. 3C). This can be accomplished by unscrewing (or otherwise uncoupling) applicator cap 708 from housing 702. Applicator cap 708 can then be placed aside.

FIG. 3C is a proximal perspective view depicting an example embodiment of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This step unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example embodiment of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example embodiment of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example embodiment of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

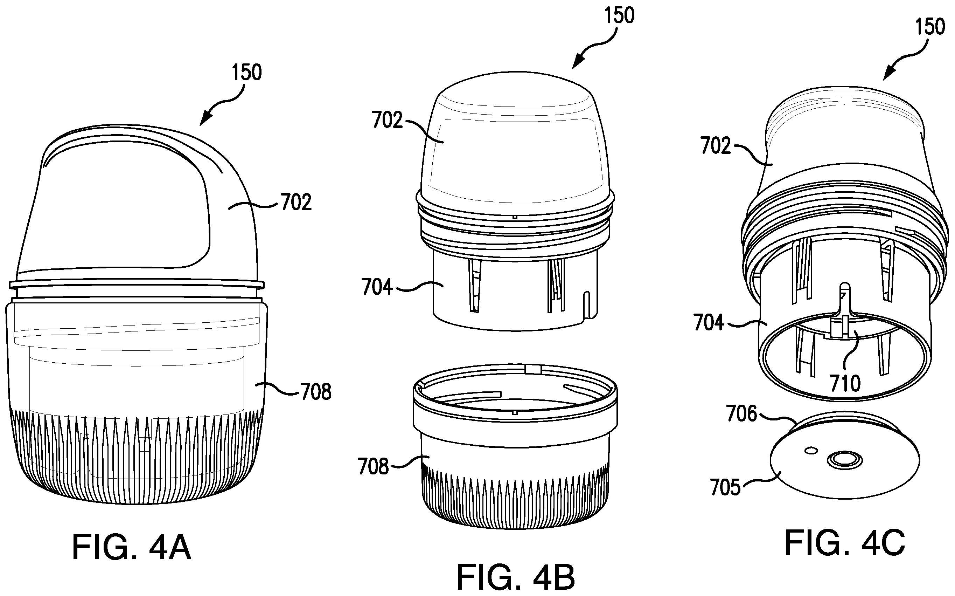

FIG. 4A is a side view depicting an example embodiment of an applicator device 150 coupled with a screw applicator cap 708. This is an example of how applicator 150 is shipped to and received by a user, prior to assembly by the user with a sensor. FIG. 4B is a side perspective view depicting applicator 150 and applicator cap 708 after being decoupled. FIG. 4C is a perspective view depicting an example embodiment of a distal end of an applicator device 150 with electronics housing 706 and adhesive patch 105 removed from the position they would have retained within sensor carrier 710 of sheath 704, when applicator cap 708 is in place.

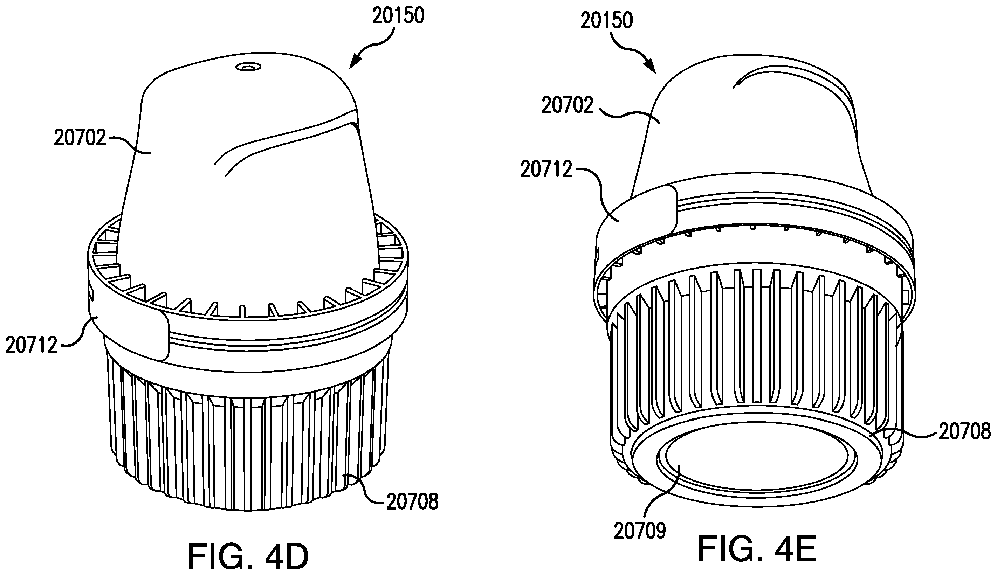

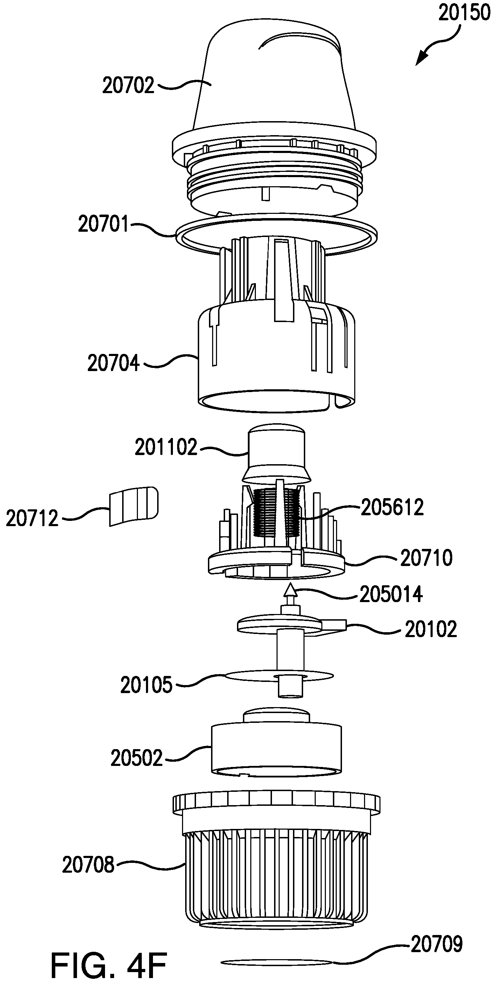

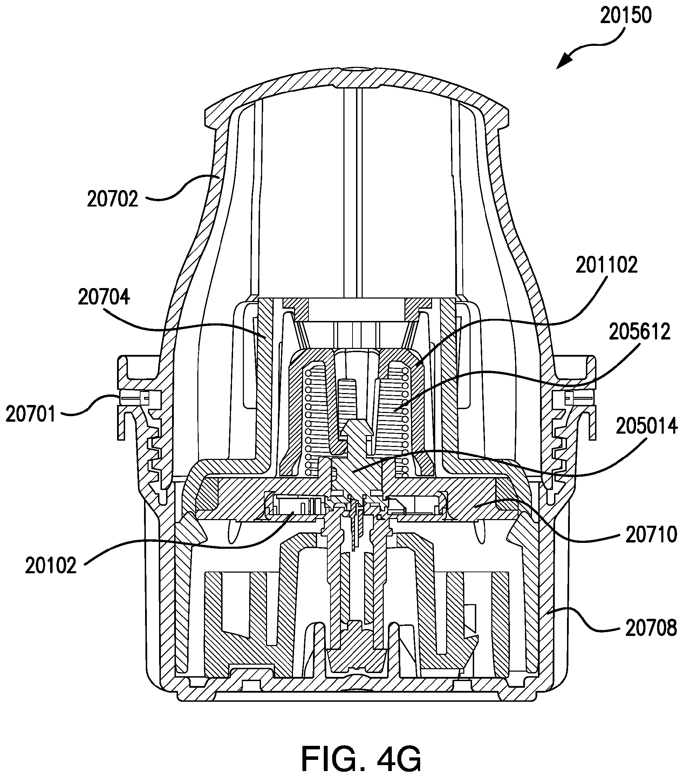

Referring to FIGs. 4D-G for purpose of illustration and not limitation, another example embodiment of an applicator device 20150 can be provided to a user as a single integrated assembly. FIGs. 4D and 4E provide perspective top and bottom views, respectively, of the applicator device 20150, FIG. 4F provides an exploded view of the applicator device 20150 and FIG. 4G provides a side cut-away view. The perspective views illustrate how applicator 20150 is shipped to and received by a user. The exploded and cut-away views illustrate the components of the applicator device 20150. The applicator device 20150 can include a housing 20702, gasket 20701, sheath 20704, sharp carrier 201102, spring 205612, sensor carrier 20710 (also referred to as a "puck carrier"), sharp hub 205014, sensor control device (also referred to as a "puck") 20102, adhesive patch 20105, desiccant 20502, applicator cap 20708, serial label 20709, and tamper evidence feature 20712. In some embodiments, as received by a user, only the housing 20702, applicator cap 20708, tamper evidence feature 20712, and label 20709 are visible. The tamper evidence feature 20712 can be, for example, a sticker coupled to each of the housing 20702 and the applicator cap 20708, and tamper evidence feature 20712 can be damaged, for example, irreparably, by uncoupling housing 20702 and applicator cap 20708, thereby indicating to a user that the housing 20702 and applicator cap 20708 have been previously uncoupled. These features are described in greater detail below.

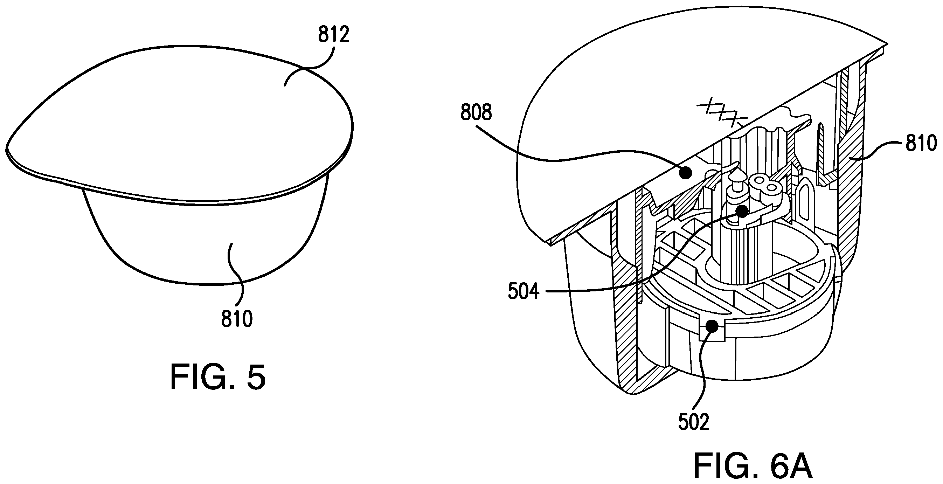

FIG. 5 is a proximal perspective view depicting an example embodiment of a tray 810 with sterilization lid 812 removably coupled thereto, which can be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

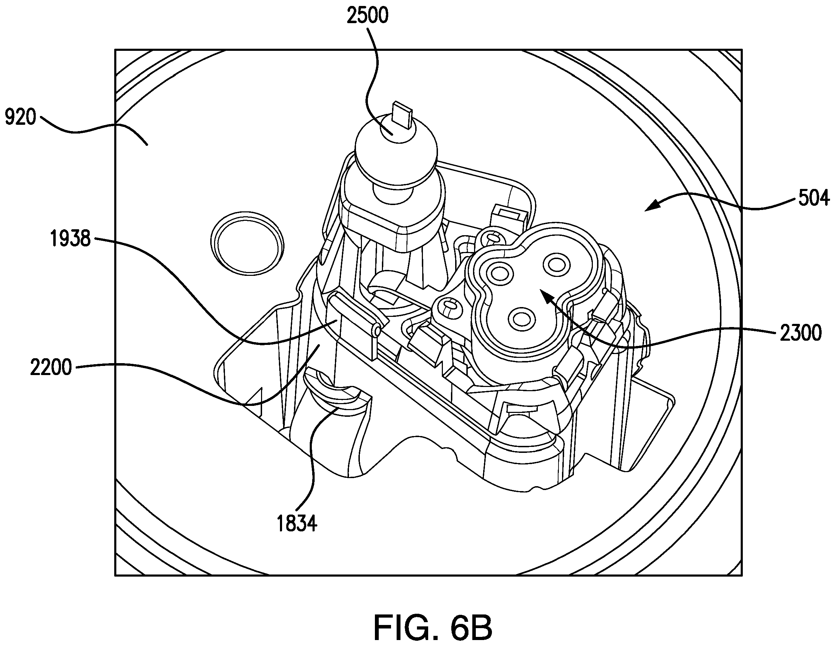

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

Referring briefly again to FIGS. 1A and 3A-3G, for the two-piece architecture system, the sensor tray 810 and the sensor applicator 150 are provided to the user as separate packages, thus requiring the user to open each package and finally assemble the system. In some applications, the discrete, sealed packages allow the sensor tray 810 and the sensor applicator 150 to be sterilized in separate sterilization processes unique to the contents of each package and otherwise incompatible with the contents of the other. More specifically, the sensor tray 810, which includes the plug assembly 207, including the sensor 104 and the sharp 220, can be sterilized using radiation sterilization, such as electron beam (or "e-beam") irradiation. Suitable radiation sterilization processes include, but are not limited to, e-beam irradiation, gamma ray irradiation, X-ray irradiation, or any combination thereof. Radiation sterilization, however, can damage the electrical components arranged within the electronics housing of the sensor control device 102. Consequently, if the sensor applicator 150, which contains the electronics housing of the sensor control device 102, needs to be sterilized, it can be sterilized via another method, such as gaseous chemical sterilization using, for example, ethylene oxide. Gaseous chemical sterilization, however, can damage the enzymes or other chemistry and biologies included on the sensor 104. Because of this sterilization incompatibility, the sensor tray 810 and the sensor applicator 150 are commonly sterilized in separate sterilization processes and subsequently packaged separately, which requires the user to finally assemble the components for use.

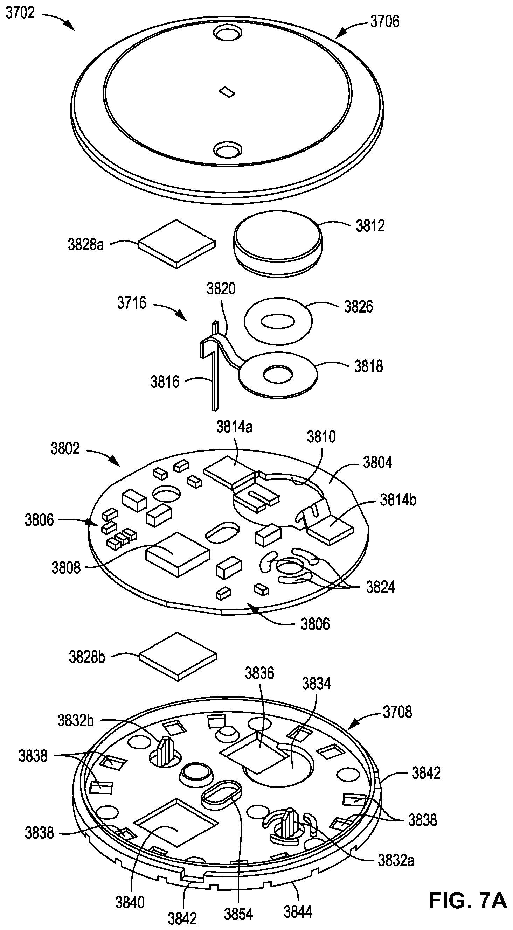

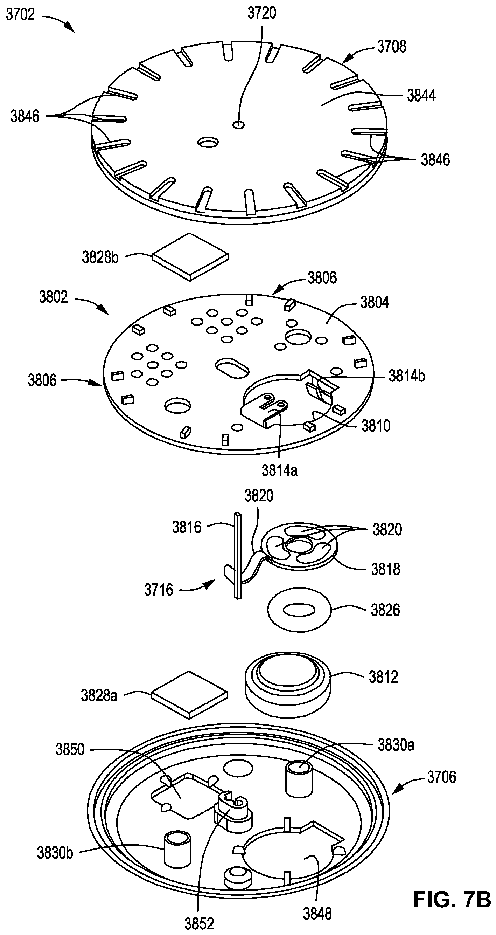

FIGS. 7A and 7B are exploded top and bottom views, respectively, of the sensor control device 3702, according to one or more embodiments. The shell 3706 and the mount 3708 operate as opposing clamshell halves that enclose or otherwise substantially encapsulate the various electronic components of the sensor control device 3702. As illustrated, the sensor control device 3702 can include a printed circuit board assembly (PCBA) 3802 that includes a printed circuit board (PCB) 3804 having a plurality of electronic modules 3806 coupled thereto. Example electronic modules 3806 include, but are not limited to, resistors, transistors, capacitors, inductors, diodes, and switches. Prior sensor control devices commonly stack PCB components on only one side of the PCB. In contrast, the PCB components 3806 in the sensor control device 3702 can be dispersed about the surface area of both sides (i.e., top and bottom surfaces) of the PCB 3804.

Besides the electronic modules 3806, the PCBA 3802 can also include a data processing unit 3808 mounted to the PCB 3804. The data processing unit 3808 can comprise, for example, an application specific integrated circuit (ASIC) configured to implement one or more functions or routines associated with operation of the sensor control device 3702. More specifically, the data processing unit 3808 can be configured to perform data processing functions, where such functions can include but are not limited to, filtering and encoding of data signals, each of which corresponds to a sampled analyte level of the user. The data processing unit 3808 can also include or otherwise communicate with an antenna for communicating with the reader device 106.

A battery aperture 3810 can be defined in the PCB 3804 and sized to receive and seat a battery 3812 configured to power the sensor control device 3702. An axial battery contact 3814a and a radial battery contact 3814b can be coupled to the PCB 3804 and extend into the battery aperture 3810 to facilitate transmission of electrical power from the battery 3812 to the PCB 3804. As their names suggest, the axial battery contact 3814a can be configured to provide an axial contact for the battery 3812, while the radial battery contact 3814b can provide a radial contact for the battery 3812. Locating the battery 3812 within the battery aperture 3810 with the battery contacts 3814a,b helps reduce the height H of the sensor control device 3702, which allows the PCB 3804 to be located centrally and its components to be dispersed on both sides (i.e., top and bottom surfaces). This also helps facilitate the chamfer 3718 provided on the electronics housing 3704.

The sensor 3716 can be centrally located relative to the PCB 3804 and include a tail 3816, a flag 3818, and a neck 3820 that interconnects the tail 3816 and the flag 3818. The tail 3816 can be configured to extend through the central aperture 3720 of the mount 3708 to be transcutaneously received beneath a user's skin. Moreover, the tail 3816 can have an enzyme or other chemistry included thereon to help facilitate analyte monitoring.

The flag 3818 can include a generally planar surface having one or more sensor contacts 3822 (three shown in FIG. 7B) arranged thereon. The sensor contact(s) 3822 can be configured to align with and engage a corresponding one or more circuitry contacts 3824 (three shown in FIG. 7A) provided on the PCB 3804. In some embodiments, the sensor contact(s) 3822 can comprise a carbon impregnated polymer printed or otherwise digitally applied to the flag 3818. Prior sensor control devices typically include a connector made of silicone rubber that encapsulates one or more compliant carbon impregnated polymer modules that serve as electrical conductive contacts between the sensor and the PCB. In contrast, the presently disclosed sensor contacts(s) 3822 provide a direct connection between the sensor 3716 and the PCB 3804 connection, which eliminates the need for the prior art connector and advantageously reduces the height H. Moreover, eliminating the compliant carbon impregnated polymer modules eliminates a significant circuit resistance and therefor improves circuit conductivity.

The sensor control device 3702 can further include a compliant member 3826, which can be arranged to interpose the flag 3818 and the inner surface of the shell 3706. More specifically, when the shell 3706 and the mount 3708 are assembled to one another, the compliant member 3826 can be configured to provide a passive biasing load against the flag 3818 that forces the sensor contact(s) 3822 into continuous engagement with the corresponding circuitry contact(s) 3824. In the illustrated embodiment, the compliant member 3826 is an elastomeric O-ring, but could alternatively comprise any other type of biasing device or mechanism, such as a compression spring or the like, without departing from the scope of the disclosure.

The sensor control device 3702 can further include one or more electromagnetic shields, shown as a first shield 3828a and a second shield The shell 3706 can provide or otherwise define a first clocking receptacle 3830a (FIG. 7B) and a second clocking receptacle 3830b (FIG. 7B), and the mount 3708 can provide or otherwise define a first clocking post 3832a (FIG. 7A) and a second clocking post 3832b (FIG. 7A). Mating the first and second clocking receptacles 3830a,b with the first and second clocking posts 3832a,b, respectively, will properly align the shell 3706 to the mount 3708.

Referring specifically to FIG. 7A, the inner surface of the mount 3708 can provide or otherwise define a plurality of pockets or depressions configured to accommodate various component parts of the sensor control device 3702 when the shell 3706 is mated to the mount 3708. For example, the inner surface of the mount 3708 can define a battery locator 3834 configured to accommodate a portion of the battery 3812 when the sensor control device 3702 is assembled. An adjacent contact pocket 3836 can be configured to accommodate a portion of the axial contact 3814a.

Moreover, a plurality of module pockets 3838 can be defined in the inner surface of the mount 3708 to accommodate the various electronic modules 3806 arranged on the bottom of the PCB 3804. Furthermore, a shield locator 3840 can be defined in the inner surface of the mount 3708 to accommodate at least a portion of the second shield 3828b when the sensor control device 3702 is assembled. The battery locator 3834, the contact pocket 3836, the module pockets 3838, and the shield locator 3840 all extend a short distance into the inner surface of the mount 3708 and, as a result, the overall height H of the sensor control device 3702 can be reduced as compared to prior sensor control devices. The module pockets 3838 can also help minimize the diameter of the PCB 3804 by allowing PCB components to be arranged on both sides (i.e., top and bottom surfaces).

Still referring to FIG. 7A, the mount 3708 can further include a plurality of carrier grip features 3842 (two shown) defined about the outer periphery of the mount 3708. The carrier grip features 3842 are axially offset from the bottom 3844 of the mount 3708, where a transfer adhesive (not shown) can be applied during assembly. In contrast to prior sensor control devices, which commonly include conical carrier grip features that intersect with the bottom of the mount, the presently disclosed carrier grip features 3842 are offset from the plane (i.e., the bottom 3844) where the transfer adhesive is applied. This can prove advantageous in helping ensure that the delivery system does not inadvertently stick to the transfer adhesive during assembly. Moreover, the presently disclosed carrier grip features 3842 eliminate the need for a scalloped transfer adhesive, which simplifies the manufacture of the transfer adhesive and eliminates the need to accurately clock the transfer adhesive relative to the mount 3708. This also increases the bond area and, therefore, the bond strength.

Referring to FIG. 7B, the bottom 3844 of the mount 3708 can provide or otherwise define a plurality of grooves 3846, which can be defined at or near the outer periphery of the mount 3708 and equidistantly spaced from each other. A transfer adhesive (not shown) can be coupled to the bottom 3844 and the grooves 3846 can be configured to help convey (transfer) moisture away from the sensor control device 3702 and toward the periphery of the mount 3708 during use. In some embodiments, the spacing of the grooves 3846 can interpose the module pockets 3838 (FIG. 7A) defined on the opposing side (inner surface) of the mount 3708. As will be appreciated, alternating the position of the grooves 3846 and the module pockets 3838 ensures that the opposing features on either side of the mount 3708 do not extend into each other. This can help maximize usage of the material for the mount 3708 and thereby help maintain a minimal height H of the sensor control device 3702. The module pockets 3838 can also significantly reduce mold sink, and improve the flatness of the bottom 3844 that the transfer adhesive bonds to.

Still referring to FIG. 7B, the inner surface of the shell 3706 can also provide or otherwise define a plurality of pockets or depressions configured to accommodate various component parts of the sensor control device 3702 when the shell 3706 is mated to the mount 3708. For example, the inner surface of the shell 3706 can define an opposing battery locator 3848 arrangeable opposite the battery locator 3834 (FIG. 7A) of the mount 3708 and configured to accommodate a portion of the battery 3812 when the sensor control device 3702 is assembled. The opposing battery locator 3848 extends a short distance into the inner surface of the shell 3706, which helps reduce the overall height H of the sensor control device 3702.

A sharp and sensor locator 3852 can also be provided by or otherwise defined on the inner surface of the shell 3706. The sharp and sensor locator 3852 can be configured to receive both the sharp (not shown) and a portion of the sensor 3716. Moreover, the sharp and sensor locator 3852 can be configured to align and/or mate with a corresponding sharp and sensor locator 2054 (FIG. 7A) provided on the inner surface of the mount 3708.

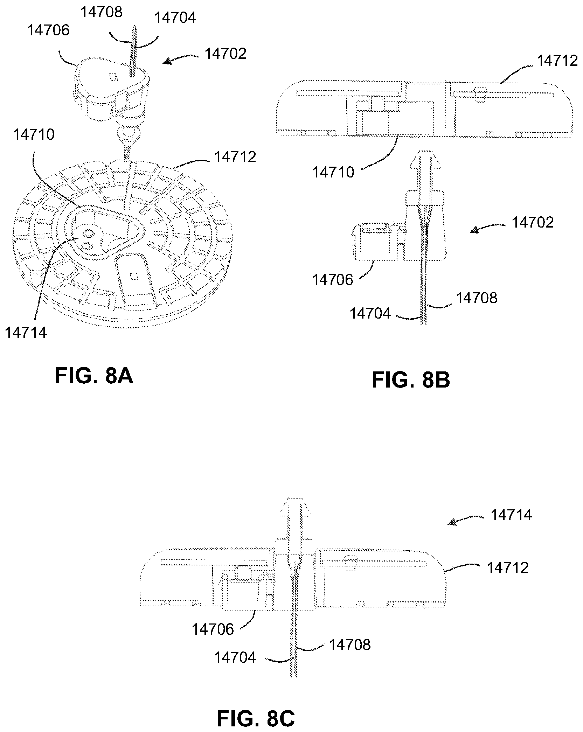

According to embodiments of the present disclosure, an alternative sensor assembly/electronics assembly connection approach is illustrated in FIGS. 8A to 8C. As shown, the sensor assembly 14702 includes sensor 14704, connector support 14706, and sharp 14708. Notably, a recess or receptacle 14710 can be defined in the bottom of the mount of the electronics assembly 14712 and provide a location where the sensor assembly 14702 can be received and coupled to the electronics assembly 14712 , and thereby fully assemble the sensor control device. The profile of the sensor assembly 14702 can match or be shaped in complementary fashion to the receptacle 14710, which includes an elastomeric sealing member 14714 (including conductive material coupled to the circuit board and aligned with the electrical contacts of the sensor 14704). Thus, when the sensor assembly 14702 is snap fit or otherwise adhered to the electronics assembly 14712 by driving the sensor assembly 14702 into the integrally formed recess 14710 in the electronics assembly 14712, the on-body device 14714 depicted in FIG. 8C is formed. This embodiment provides an integrated connector for the sensor assembly 14702 within the electronics assembly 14712.

Additional information regarding sensor assemblies is provided in U.S. Publication No. 2013/0150691 and U.S. Publication No. 2021/0204841.

According to embodiments of the present disclosure, the sensor control device 102 can be modified to provide a one-piece architecture that can be subjected to sterilization techniques specifically designed for a one-piece architecture sensor control device. A one-piece architecture allows the sensor applicator 150 and the sensor control device 102 to be shipped to the user in a single, sealed package that does not require any final user assembly steps. Rather, the user need only open one package and subsequently deliver the sensor control device 102 to the target monitoring location. The one-piece system architecture described herein can prove advantageous in eliminating component parts, various fabrication process steps, and user assembly steps. As a result, packaging and waste are reduced, and the potential for user error or contamination to the system is mitigated.

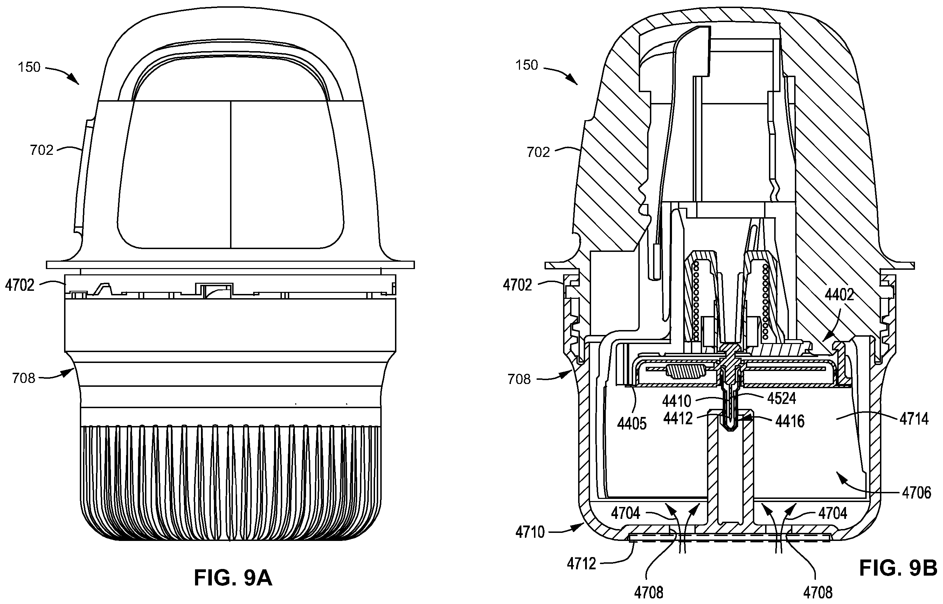

FIGS. 9A and 9B are side and cross-sectional side views, respectively, of an example embodiment of the sensor applicator 150 with the applicator cap 708 coupled thereto. More specifically, FIG. 9A depicts how the sensor applicator 150 might be shipped to and received by a user, and FIG. 9B depicts the sensor control device 4402 arranged within the sensor applicator 150. Accordingly, the fully assembled sensor control device 4402 can already be assembled and installed within the sensor applicator 150 prior to being delivered to the user, thus removing any additional assembly steps that a user would otherwise have to perform.

The fully assembled sensor control device 4402 can be loaded into the sensor applicator 150, and the applicator cap 708 can subsequently be coupled to the sensor applicator 150. In some embodiments, the applicator cap 708 can be threaded to the housing 702 and include a tamper ring 4702. Upon rotating (e.g., unscrewing) the applicator cap 708 relative to the housing 702, the tamper ring 4702 can shear and thereby free the applicator cap 708 from the sensor applicator 150.

According to the present disclosure, while loaded in the sensor applicator 150, the sensor control device 4402 can be subjected to gaseous chemical sterilization 4704 configured to sterilize the electronics housing 4404 and any other exposed portions of the sensor control device 4402. To accomplish this, a chemical can be injected into a sterilization chamber 4706 cooperatively defined by the sensor applicator 150 and the interconnected cap 210. In some applications, the chemical can be injected into the sterilization chamber 4706 via one or more vents 4708 defined in the applicator cap 708 at its proximal end 610. Example chemicals that can be used for the gaseous chemical sterilization 4704 include, but are not limited to, ethylene oxide, vaporized hydrogen peroxide, nitrogen oxide (e.g., nitrous oxide, nitrogen dioxide, etc.), and steam.

Since the distal portions of the sensor 4410 and the sharp 4412 are sealed within the sensor cap 4416, the chemicals used during the gaseous chemical sterilization process do not interact with the enzymes, chemistry, and biologics provided on the tail 4524 and other sensor components, such as membrane coatings that regulate analyte influx.

Once a desired sterility assurance level has been achieved within the sterilization chamber 4706, the gaseous solution can be removed and the sterilization chamber 4706 can be aerated. Aeration can be achieved by a series of vacuums and subsequently circulating a gas (e.g., nitrogen) or filtered air through the sterilization chamber 4706. Once the sterilization chamber 4706 is properly aerated, the vents 4708 can be occluded with a seal 4712 (shown in dashed lines).

In some embodiments, the seal 4712 can comprise two or more layers of different materials. The first layer can be made of a synthetic material (e.g., a flash-spun high-density polyethylene fiber), such as Tyvek® available from DuPont®. Tyvek® is highly durable and puncture resistant and allows the permeation of vapors. The Tyvek® layer can be applied before the gaseous chemical sterilization process, and following the gaseous chemical sterilization process, a foil or other vapor and moisture resistant material layer can be sealed (e.g., heat sealed) over the Tyvek® layer to prevent the ingress of contaminants and moisture into the sterilization chamber 4706. In other embodiments, the seal 4712 can comprise only a single protective layer applied to the applicator cap 708. In such embodiments, the single layer can be gas permeable for the sterilization process, but can also be capable of protection against moisture and other harmful elements once the sterilization process is complete.

With the seal 4712 in place, the applicator cap 708 provides a barrier against outside contamination, and thereby maintains a sterile environment for the assembled sensor control device 4402 until the user removes (unthreads) the applicator cap 708. The applicator cap 708 can also create a dust-free environment during shipping and storage that prevents the adhesive patch 4714 from becoming dirty.

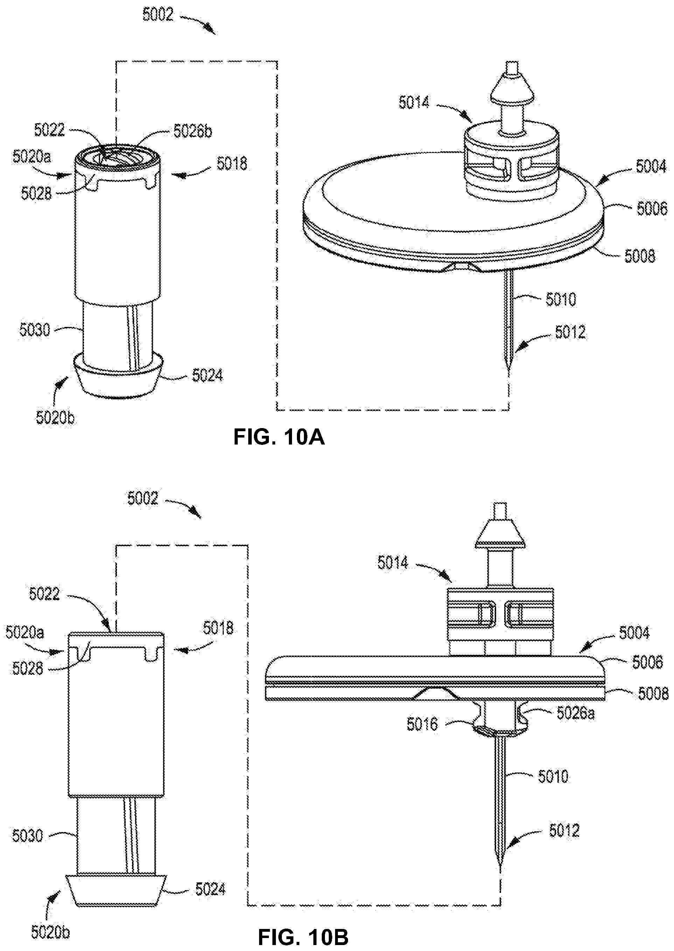

FIGS. 10A and 10B are isometric and side views, respectively, of another example sensor control device 5002, according to one or more embodiments of the present disclosure. The sensor control device 5002 can be similar in some respects to the sensor control device 102 of FIG. 1A and therefore can be best understood with reference thereto. Moreover, the sensor control device 5002 can replace the sensor control device 102 of FIG. 1A and, therefore, can be used in conjunction with the sensor applicator 150 of FIG. 1A, which can deliver the sensor control device 5002 to a target monitoring location on a user's skin.

Unlike the sensor control device 102 of FIG. 1A, however, the sensor control device 5002 can comprise a one-piece system architecture not requiring a user to open multiple packages and finally assemble the sensor control device 5002 prior to application. Rather, upon receipt by the user, the sensor control device 5002 can already be fully assembled and properly positioned within the sensor applicator 150 (FIG. 1A). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and can have a circular cross-section. In other embodiments, however, the electronics housing 5004 can exhibit other cross-sectional shapes, such as ovoid or polygonal, without departing from the scope of the disclosure. The electronics housing 5004 can be configured to house or otherwise contain various electrical components used to operate the sensor control device 5002. In at least one embodiment, an adhesive patch (not shown) can be arranged at the bottom of the electronics housing 5004. The adhesive patch can be similar to the adhesive patch 105 of FIG. 1A, and can thus help adhere the sensor control device 5002 to the user's skin for use.

As illustrated, the sensor control device 5002 includes an electronics housing 5004 that includes a shell 5006 and a mount 5008 that is mateable with the shell 5006. The shell 5006 can be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 can be secured to the mount 5008 such that a sealed interface is generated therebetween.

The sensor control device 5002 can further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 can include a sharp hub 5014 configured to secure and carry the sharp 5012. As best seen in FIG. 10B, the sharp hub 5014 can include or otherwise define a mating member 5016. To couple the sharp 5012 to the sensor control device 5002, the sharp 5012 can be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 can be received within a hollow or recessed (arcuate) portion of the sharp 5012. The remaining portion of the sensor 5010 is arranged within the interior of the electronics housing 5004.

The sensor control device 5002 can further include a sensor cap 5018, shown exploded or detached from the electronics housing 5004 in FIGS. 10A-10B. The sensor cap 5018 can be removably coupled to the sensor control device 5002 (e.g., the electronics housing 5004) at or near the bottom of the mount 5008. The sensor cap 5018 can help provide a sealed barrier that surrounds and protects the exposed portions of the sensor 5010 and the sharp 5012 from gaseous chemical sterilization. As illustrated, the sensor cap 5018 can comprise a generally cylindrical body having a first end 5020a and a second end 5020b opposite the first end 5020a. The first end 5020a can be open to provide access into an inner chamber 5022 defined within the body. In contrast, the second end 5020b can be closed and can provide or otherwise define an engagement feature 5024. As described herein, the engagement feature 5024 can help mate the sensor cap 5018 to the cap (e.g., the applicator cap 708 of FIG. 3B) of a sensor applicator (e.g., the sensor applicator 150 of FIGS. 1 and 3A-3G), and can help remove the sensor cap 5018 from the sensor control device 5002 upon removing the cap from the sensor applicator 150.

The sensor cap 5018 can be removably coupled to the electronics housing 5004 at or near the bottom of the mount 5008. More specifically, the sensor cap 5018 can be removably coupled to the mating member 5016, which extends distally from the bottom of the mount 5008.

In at least one embodiment, for example, the mating member 5016 can define a set of external threads 5026a (FIG. 10B) mateable with a set of internal threads 5026b (FIG. 10A) defined by the sensor cap 5018. In some embodiments, the external and internal threads 5026a, b can comprise a flat thread design (e.g., lack of helical curvature), which can prove advantageous in molding the parts. Alternatively, the external and internal threads 5026a,b can comprise a helical threaded engagement. Accordingly, the sensor cap 5018 can be threadably coupled to the sensor control device 5002 at the mating member 5016 of the sharp hub 5014. In other embodiments, the sensor cap 5018 can be removably coupled to the mating member 5016 via other types of engagements including, but not limited to, an interference or friction fit, or a frangible member or substance that can be broken with minimal separation force (e.g., axial or rotational force).

In some embodiments, the sensor cap 5018 can comprise a monolithic (singular) structure extending between the first and second ends 5020a, b. In other embodiments, however, the sensor cap 5018 can comprise two or more component parts. In the illustrated embodiment, for example, the sensor cap 5018 can include a seal ring 5028 positioned at the first end 5020a and a desiccant cap 5030 arranged at the second end 5020b. The seal ring 5028 can be configured to help seal the inner chamber 5022, as described in more detail below. In at least one embodiment, the seal ring 5028 can comprise an elastomeric O-ring. The desiccant cap 5030 can house or comprise a desiccant to help maintain preferred humidity levels within the inner chamber 5022. The desiccant cap 5030 can also define or otherwise provide the engagement feature 5024 of the sensor cap 5018.

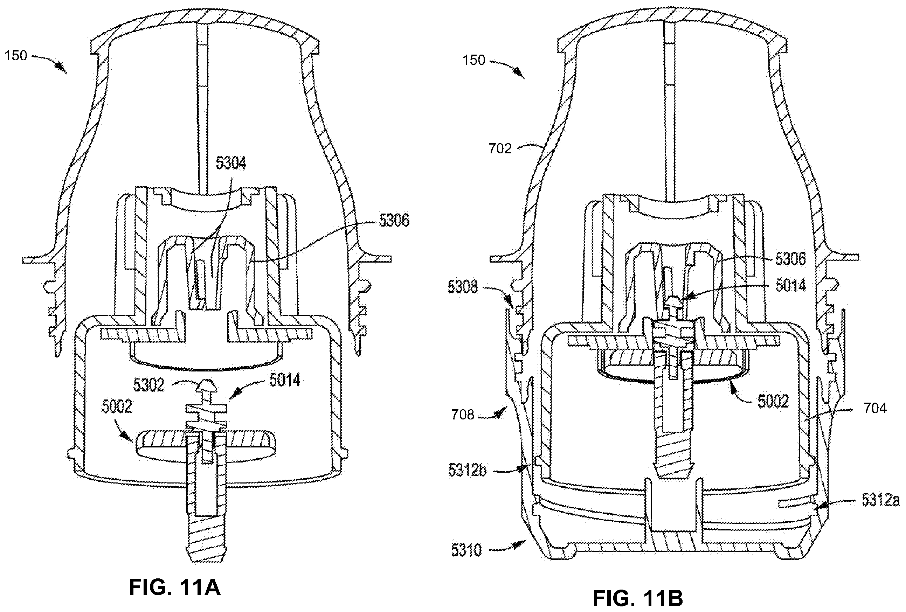

FIGS. 11A-11C are progressive cross-sectional side views showing assembly of the sensor applicator 150 with the sensor control device 5002, according to one or more embodiments. Once the sensor control device 5002 is fully assembled, it can then be loaded into the sensor applicator 150. With reference to FIG. 11A, the sharp hub 5014 can include or otherwise define a hub snap pawl 5302 configured to help couple the sensor control device 5002 to the sensor applicator 150. More specifically, the sensor control device 5002 can be advanced into the interior of the sensor applicator 150 and the hub snap pawl 5302 can be received by corresponding arms 5304 of a sharp carrier 5306 positioned within the sensor applicator 150.

In FIG. 11B, the sensor control device 5002 is shown received by the sharp carrier 5306 and, therefore, secured within the sensor applicator 150. Once the sensor control device 5002 is loaded into the sensor applicator 150, the applicator cap 708 can be coupled to the sensor applicator 150. In some embodiments, the applicator cap 708 and the housing 702 can have opposing, mateable sets of threads 5308 that enable the applicator cap 708 to be screwed onto the housing 702 in a clockwise (or counter-clockwise) direction and thereby secure the applicator cap 708 to the sensor applicator 150.

As illustrated, the sheath 704 is also positioned within the sensor applicator 150, and the sensor applicator 150 can include a sheath locking mechanism 5310 configured to ensure that the sheath 704 does not prematurely collapse during a shock event. In the illustrated embodiment, the sheath locking mechanism 5310 can comprise a threaded engagement between the applicator cap 708 and the sheath 704. More specifically, one or more internal threads 5312a can be defined or otherwise provided on the inner surface of the applicator cap 708, and one or more external threads 5312b can be defined or otherwise provided on the sheath 704. The internal and external threads 5312a,b can be configured to threadably mate as the applicator cap 708 is threaded to the sensor applicator 150 at the threads 5308. The internal and external threads 5312a,b can have the same thread pitch as the threads 5308 that enable the applicator cap 708 to be screwed onto the housing 702.

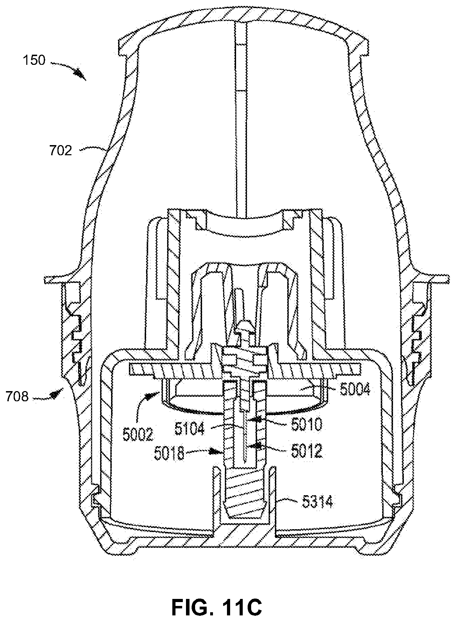

In FIG. 11C, the applicator cap 708 is shown fully threaded (coupled) to the housing 702. As illustrated, the applicator cap 708 can further provide and otherwise define a cap post 5314 centrally located within the interior of the applicator cap 708 and extending proximally from the bottom thereof. The cap post 5314 can be configured to receive at least a portion of the sensor cap 5018 as the applicator cap 708 is screwed onto the housing 702.

With the sensor control device 5002 loaded within the sensor applicator 150 and the applicator cap 708 properly secured, the sensor control device 5002 can then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the tail 5104, and other sensor components, such as membrane coatings that regulate analyte influx.

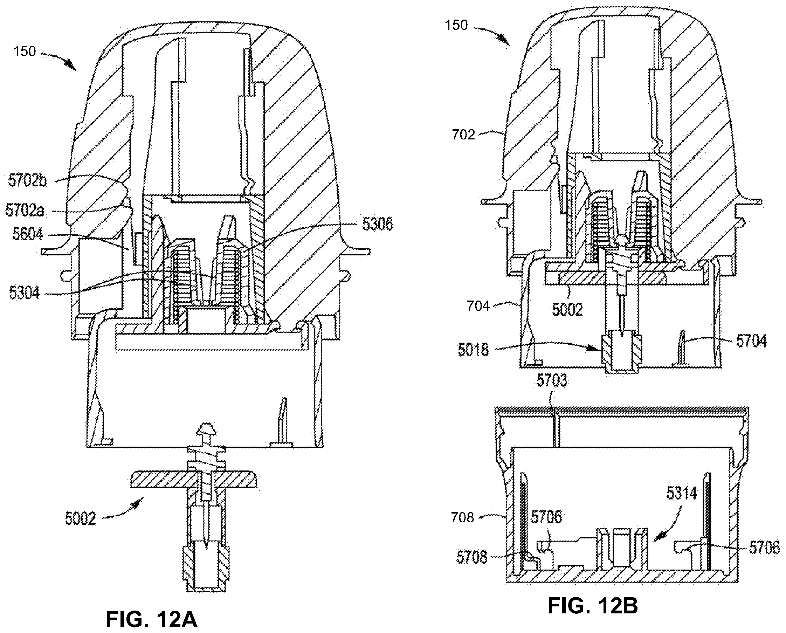

FIGS. 12A-12C are progressive cross-sectional side views showing assembly and disassembly of an alternative embodiment of the sensor applicator 150 with the sensor control device 5002, according to one or more additional embodiments. A fully assembled sensor control device 5002 can be loaded into the sensor applicator 150 by coupling the hub snap pawl 5302 into the arms 5304 of the sharp carrier 5306 positioned within the sensor applicator 150, as generally described above.

In the illustrated embodiment, the sheath arms 5604 of the sheath 704 can be configured to interact with a first detent 5702a and a second detent 5702b defined within the interior of the housing 702. The first detent 5702a can alternately be referred to a "locking" detent, and the second detent 5702b can alternately be referred to as a "firing" detent. When the sensor control device 5002 is initially installed in the sensor applicator 150, the sheath arms 5604 can be received within the first detent 5702a. As discussed below, the sheath 704 can be actuated to move the sheath arms 5604 to the second detent 5702b, which places the sensor applicator 150 in firing position.

In FIG. 12B, the applicator cap 708 is aligned with the housing 702 and advanced toward the housing 702 so that the sheath 704 is received within the applicator cap 708. Instead of rotating the applicator cap 708 relative to the housing 702, the threads of the applicator cap 708 can be snapped onto the corresponding threads of the housing 702 to couple the applicator cap 708 to the housing 702. Axial cuts or slots 5703 (one shown) defined in the applicator cap 708 can allow portions of the applicator cap 708 near its threading to flex outward to be snapped into engagement with the threading of the housing 702. As the applicator cap 708 is snapped to the housing 702, the sensor cap 5018 can correspondingly be snapped into the cap post 5314.

Similar to the embodiment of FIGS. 11A-11C, the sensor applicator 150 can include a sheath locking mechanism configured to ensure that the sheath 704 does not prematurely collapse during a shock event. In the illustrated embodiment, the sheath locking mechanism includes one or more ribs 5704 (one shown) defined near the base of the sheath 704 and configured to interact with one or more ribs 5706 (two shown) and a shoulder 5708 defined near the base of the applicator cap 708. The ribs 5704 can be configured to inter-lock between the ribs 5706 and the shoulder 5708 while attaching the applicator cap 708 to the housing 702. More specifically, once the applicator cap 708 is snapped onto the housing 702, the applicator cap 708 can be rotated (e.g., clockwise), which locates the ribs 5704 of the sheath 704 between the ribs 5706 and the shoulder 5708 of the applicator cap 708 and thereby "locks" the applicator cap 708 in place until the user reverse rotates the applicator cap 708 to remove the applicator cap 708 for use. Engagement of the ribs 5704 between the ribs 5706 and the shoulder 5708 of the applicator cap 708 can also prevent the sheath 704 from collapsing prematurely.

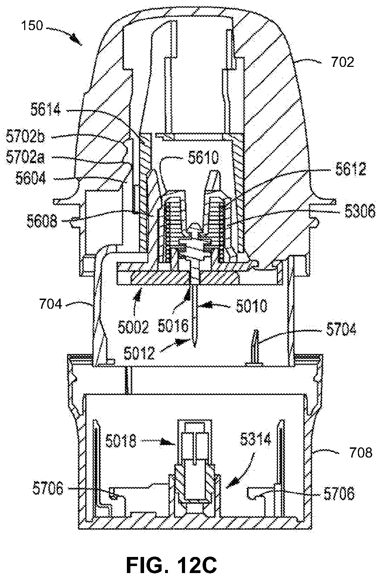

In FIG. 12C, the applicator cap 708 is removed from the housing 702. As with the embodiment of FIGS. 12A-12C, the applicator cap 708 can be removed by reverse rotating the applicator cap 708, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 708 is unscrewed from the housing 702, the ribs 5704 defined on the sheath 704 can slidingly engage the tops of the ribs 5706 defined on the applicator cap 708. The tops of the ribs 5706 can provide corresponding ramped surfaces that result in an upward displacement of the sheath 704 as the applicator cap 708 is rotated, and moving the sheath 704 upward causes the sheath arms 5604 to flex out of engagement with the first detent 5702a to be received within the second detent 5702b. As the sheath 704 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 702, the mating member 5016 can correspondingly retract until it becomes flush, substantially flush, or sub-flush with the bottom of the sensor control device 5002. At this point, the sensor applicator 150 in firing position. Accordingly, in this embodiment, removing the applicator cap 708 correspondingly causes the mating member 5016 to retract.

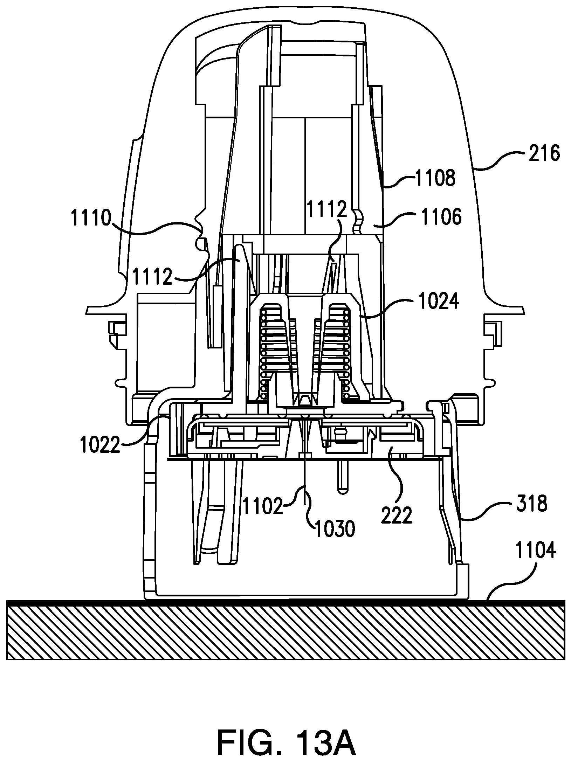

FIGS. 13A-13F illustrate example details of embodiments of the internal device mechanics of "firing" the applicator 150 to apply sensor control device 102 to a user and including retracting sharp 1030 safely back into used applicator 150. All together, these drawings represent an example sequence of driving sharp 1030 (supporting a sensor coupled to sensor control device 102) into the skin of a user, withdrawing the sharp while leaving the sensor behind in operative contact with interstitial fluid of the user, and adhering the sensor control device to the skin of the user with an adhesive. Modification of such activity for use with the alternative applicator assembly embodiments and components can be appreciated in reference to the same by those with skill in the art. Moreover, applicator 150 can be a sensor applicator having one-piece architecture or a two-piece architecture as disclosed herein.

Turning now to FIG. 13A, a sensor 1102 is supported within sharp 1030, just above the skin 1104 of the user. Rails 1106 (optionally three of them) of an upper guide section 1108 can be provided to control applicator 150 motion relative to sheath 704. The sheath 704 is held by detent features 1110 within the applicator 150 such that appropriate downward force along the longitudinal axis of the applicator 150 will cause the resistance provided by the detent features 1110 to be overcome so that sharp 1030 and sensor control device 102 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 102.

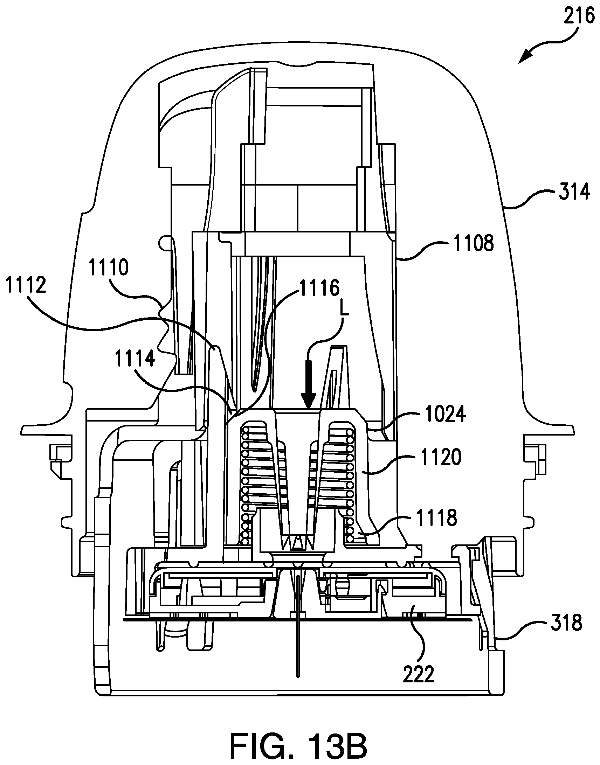

In FIG. 13B, user force is applied to overcome or override detent features 1110 and sheath 704 collapses into housing 702 driving the sensor control device 102 (with associated parts) to translate down as indicated by the arrow L along the longitudinal axis. An inner diameter of the upper guide section 1108 of the sheath 704 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized. According to embodiments, rather than employing user force to drive the sensor control device 102 to translate down as indicated by the arrow L along the longitudinal axis, housing 702 can include a button (for example, not limitation, a push button) which activates a drive spring (for example, not limitation, a coil spring) to drive the sensor control device 102.

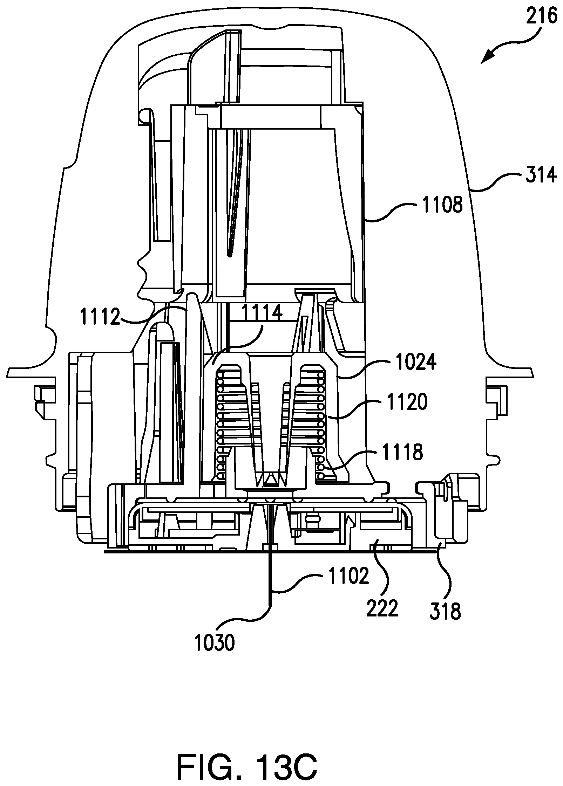

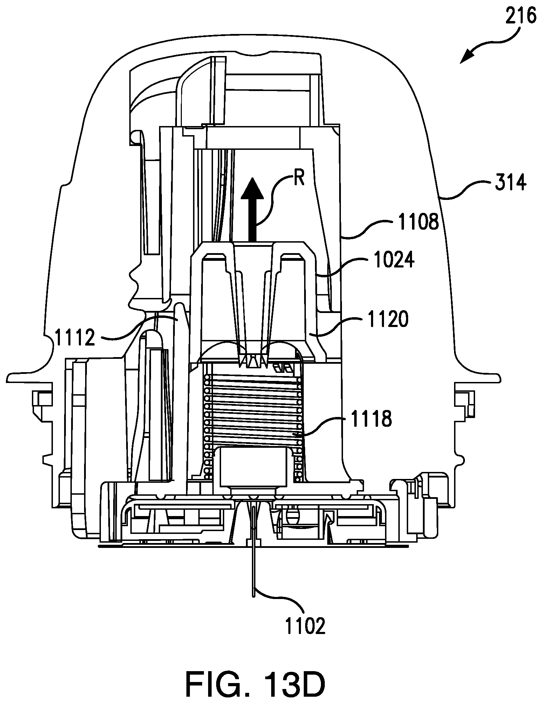

In FIG. 13C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 13D.

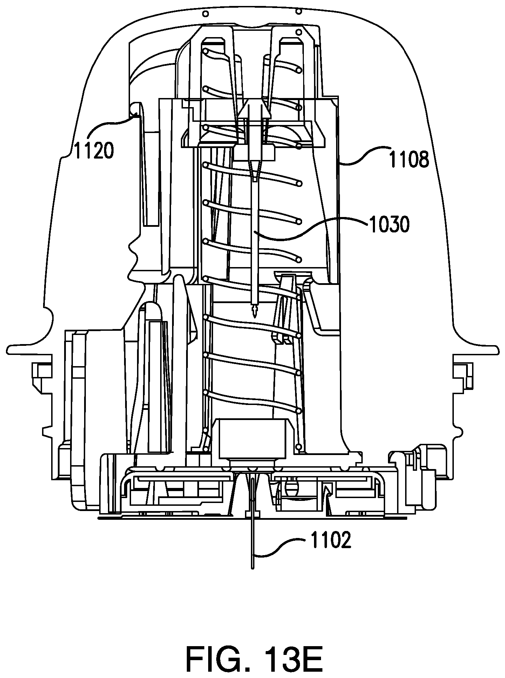

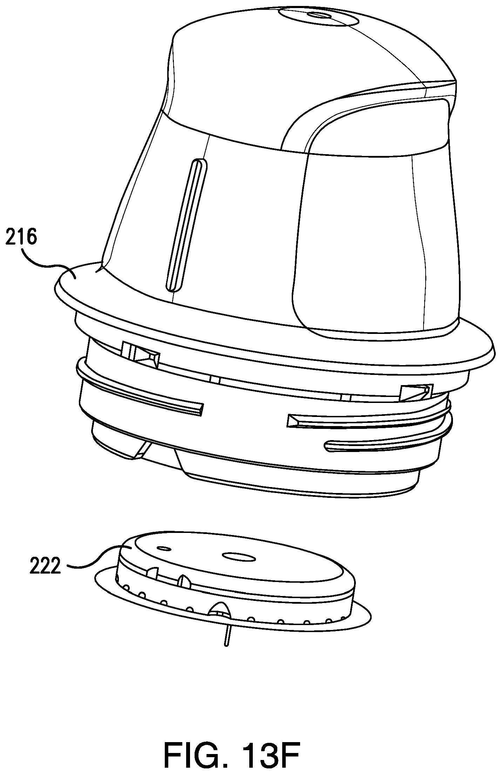

With the sharp 1030 fully retracted as shown in FIG. 13E, the upper guide section 1108 of the sheath 704 is set with a final locking feature 1120. As shown in FIG. 13F, the spent applicator assembly 150 is removed from the insertion site, leaving behind the sensor control device 102, and with the sharp 1030 secured safely inside the applicator assembly 150. The spent applicator assembly 150 is now ready for disposal.

Operation of the applicator 150 when applying the sensor control device 102 is designed to provide the user with a sensation that both the insertion and retraction of the sharp 1030 is performed automatically by the internal mechanisms of the applicator 150. In other words, the present disclosure avoids the user experiencing the sensation that he is manually driving the sharp 1030 into his skin. Thus, once the user applies sufficient force to overcome the resistance from the detent features of the applicator 150, the resulting actions of the applicator 150 are perceived to be an automated response to the applicator being "triggered." The user does not perceive that he is supplying additional force to drive the sharp 1030 to pierce his skin despite that all the driving force is provided by the user and no additional biasing/driving means are used to insert the sharp 1030. As detailed above in FIG. 13C, the retraction of the sharp 1030 is automated by the coil return spring 1118 of the applicator 150.

With respect to any of the applicator embodiments described herein, as well as any of the components thereof, including but not limited to the sharp, sharp module and sensor module embodiments, those of skill in the art will understand that said embodiments can be dimensioned and configured for use with sensors configured to sense an analyte level in a bodily fluid in the epidermis, dermis, or subcutaneous tissue of a subject. In some embodiments, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator embodiments, those of skill in the art will appreciate that certain embodiments of sharps can be dimensioned and configured to be positioned at a different end-depth in the subject's body relative to the final end-depth of the analyte sensor. In some embodiments, for example, a sharp can be positioned at a first end-depth in the subject's epidermis prior to retraction, while a distal portion of an analyte sensor can be positioned at a second end-depth in the subject's dermis. In other embodiments, a sharp can be positioned at a first end-depth in the subject's dermis prior to retraction, while a distal portion of an analyte sensor can be positioned at a second end-depth in the subject's subcutaneous tissue. In still other embodiments, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator embodiments described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator embodiments, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator embodiments, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019. Further details regarding embodiments of applicators, their components, and variants thereof, are described in U.S. Patent Publication Nos. 2013/0150691, 2016/0331283, and 2018/023552. Further details regarding embodiments of sharp modules, sharps, their components, and variants thereof, are described in U.S. Patent Publication No. 2014/0171771.

Biochemical sensors can be described by one or more sensing characteristics. A common sensing characteristic is referred to as the biochemical sensor's sensitivity, which is a measure of the sensor's responsiveness to the concentration of the chemical or composition it is designed to detect. For electrochemical sensors, this response can be in the form of an electrical current (amperometric) or electrical charge (coulometric). For other types of sensors, the response can be in a different form, such as a photonic intensity (e.g., optical light). The sensitivity of a biochemical analyte sensor can vary depending on a number of factors, including whether the sensor is in an *in vitro* state or an *in vivo* state.

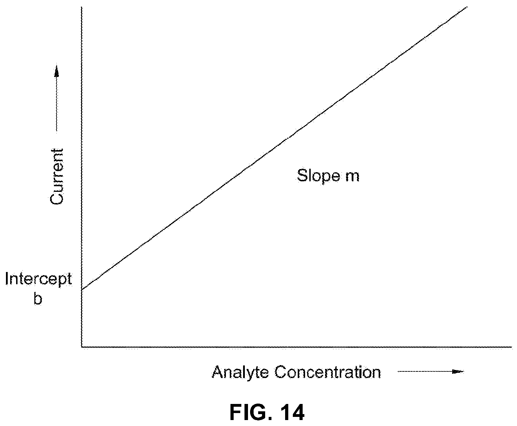

FIG. 14 is a graph depicting the *in vitro* sensitivity of an amperometric analyte sensor. The *in vitro* sensitivity can be obtained by *in vitro* testing the sensor at various analyte concentrations and then performing a regression (e.g., linear or non-linear) or other curve fitting on the resulting data. In this example, the analyte sensor's sensitivity is linear, or substantially linear, and can be modeled according to the equation $y=mx+b$, where $y$ is the sensor's electrical output current, $x$ is the analyte level (or concentration), $m$ is the slope of the sensitivity and $b$ is the intercept of the sensitivity, where the intercept generally corresponds to a background signal (e.g., noise). For sensors with a linear or substantially linear response, the analyte level that corresponds to a given current can be determined from the slope and intercept of the sensitivity. Sensors with a non-linear sensitivity require additional information to determine the analyte level resulting from the sensor's output current, and those of ordinary skill in the art are familiar with manners by which to model non-linear sensitivities. In certain embodiments of *in vivo* sensors, the *in vitro* sensitivity can be the same as the *in vivo* sensitivity, but in other embodiments a transfer (or conversion) function is used to translate the *in vitro* sensitivity into the *in vivo* sensitivity that is applicable to the sensor's intended *in vivo* use.

Calibration is a technique for improving or maintaining accuracy by adjusting a sensor's measured output to reduce the differences with the sensor's expected output. One or more parameters that describe the sensor's sensing characteristics, like its sensitivity, are established for use in the calibration adjustment.

Certain *in vivo* analyte monitoring systems require calibration to occur after implantation of the sensor into the user or patient, either by user interaction or by the system itself in an automated fashion. For example, when user interaction is required, the user performs an *in vitro* measurement (e.g., a blood glucose (BG) measurement using a finger stick and an *in vitro* test strip) and enters this into the system, while the analyte sensor is implanted. The system then compares the *in vitro* measurement with the *in vivo* signal and, using the differential, determines an estimate of the sensor's *in vivo* sensitivity. The *in vivo* sensitivity can then be used in an algorithmic process to transform the data collected with the sensor to a value that indicates the user's analyte level. This and other processes that require user action to perform calibration are referred to as "user calibration." Systems can require user calibration due to instability of the sensor's sensitivity, such that the sensitivity drifts or changes over time. Thus, multiple user calibrations (e.g., according to a periodic (e.g., daily) schedule, variable schedule, or on an as-needed basis) can be required to maintain accuracy. While the embodiments described herein can incorporate a degree of user calibration for a particular implementation, generally this is not preferred as it requires the user to perform a painful or otherwise burdensome BG measurement, and can introduce user error.

Some *in vivo* analyte monitoring systems can regularly adjust the calibration parameters through the use of automated measurements of characteristics of the sensor made by the system itself (e.g., processing circuitry executing software). The repeated adjustment of the sensor's sensitivity based on a variable measured by the system (and not the user) is referred to generally as "system" (or automated) calibration, and can be performed with user calibration, such as an early BG measurement, or without user calibration. Like the case with repeated user calibrations, repeated system calibrations are typically necessitated by drift in the sensor's sensitivity over time. Thus, while the embodiments described herein can be used with a degree of automated system calibration, preferably the sensor's sensitivity is relatively stable over time such that post-implantation calibration is not required.

Some *in vivo* analyte monitoring systems operate with a sensor that is factory calibrated. Factory calibration refers to the determination or estimation of the one or more calibration parameters prior to distribution to the user or healthcare professional (HCP). The calibration parameter can be determined by the sensor manufacturer (or the manufacturer of the other components of the sensor control device if the two entities are different). Many *in vivo* sensor manufacturing processes fabricate the sensors in groups or batches referred to as production lots, manufacturing stage lots, or simply lots. A single lot can include thousands of sensors.

Sensors can include a calibration code or parameter which can be derived or determined during one or more sensor manufacturing processes and coded or programmed, as part of the manufacturing process, in the data processing device of the analyte monitoring system or provided on the sensor itself, for example, as a bar code, a laser tag, an RFID tag, or other machine readable information provided on the sensor. User calibration during *in vivo* use of the sensor can be obviated, or the frequency of *in vivo* calibrations during sensor wear can be reduced if the code is provided to a receiver (or other data processing device). In embodiments where the calibration code or parameter is provided on the sensor itself, prior to or at the start of the sensor use, the calibration code or parameter can be automatically transmitted or provided to the data processing device in the analyte monitoring system.

Some *in vivo* analyte monitoring system operate with a sensor that can be one or more of factory calibrated, system calibrated, and/or user calibrated. For example, the sensor can be provided with a calibration code or parameter which can allow for factory calibration. If the information is provided to a receiver (for example, entered by a user), the sensor can operate as a factory calibrated sensor. If the information is not provided to a receiver, the sensor can operate as a user calibrated sensor and/or a system calibrated sensor.

In a further aspect, programming or executable instructions can be provided or stored in the data processing device of the analyte monitoring system, and/or the receiver/controller unit, to provide a time varying adjustment algorithm to the *in vivo* sensor during use. For example, based on a retrospective statistical analysis of analyte sensors used *in vivo* and the corresponding glucose level feedback, a predetermined or analytical curve or a database can be generated which is time based, and configured to provide additional adjustment to the one or more *in vivo* sensor parameters to compensate for potential sensor drift in stability profile, or other factors.

In accordance with the disclosed subject matter, the analyte monitoring system can be configured to compensate or adjust for the sensor sensitivity based on a sensor drift profile. A time varying parameter $\beta(t)$ can be defined or determined based on analysis of sensor behavior during *in vivo* use, and a time varying drift profile can be determined. In certain aspects, the compensation or adjustment to the sensor sensitivity can be programmed in the receiver unit, the controller or data processor of the analyte monitoring system such that the compensation or the adjustment or both can be performed automatically and/or iteratively when sensor data is received from the analyte sensor. In accordance with the disclosed subject matter, the adjustment or compensation algorithm can be initiated or executed by the user (rather than self-initiating or executing) such that the adjustment or the compensation to the analyte sensor sensitivity profile is performed or executed upon user initiation or activation of the corresponding function or routine, or upon the user entering the sensor calibration code.

In accordance with the disclosed subject matter, each sensor in the sensor lot (in some instances not including sample sensors used for *in vitro* testing) can be examined non-destructively to determine or measure its characteristics such as membrane thickness at one or more points of the sensor, and other characteristics including physical characteristics such as the surface area/volume of the active area can be measured or determined. Such measurement or determination can be performed in an automated manner using, for example, optical scanners or other suitable measurement devices or systems, and the determined sensor characteristics for each sensor in the sensor lot is compared to the corresponding mean values based on the sample sensors for possible correction of the calibration parameter or code assigned to each sensor. For example, for a calibration parameter defined as the sensor sensitivity, the sensitivity is approximately inversely proportional to the membrane thickness, such that, for example, a sensor having a measured membrane thickness of approximately 4% greater than the mean membrane thickness for the sampled sensors from the same sensor lot as the sensor, the sensitivity assigned to that sensor in one embodiment is the mean sensitivity determined from the sampled sensors divided by 1.04. Likewise, since the sensitivity is approximately proportional to active area of the sensor, a sensor having measured active area of approximately 3% lower than the mean active area for the sampled sensors from the same sensor lot, the sensitivity assigned to that sensor is the mean sensitivity multiplied by 0.97. The assigned sensitivity can be determined from the mean sensitivity from the sampled sensors, by multiple successive adjustments for each examination or measurement of the sensor. In certain embodiments, examination or measurement of each sensor can additionally include measurement of membrane consistency or texture in addition to the membrane thickness and/or surface are or volume of the active sensing area.

Additional information regarding sensor calibration is provided in U.S. Publication No. 2010/0230285 and U.S. Publication No. 2019/0274598.

The storage memory 5030 of the sensor control device 102 can include the software blocks related to communication protocols of the communication module. For example, the storage memory 5030 can include a BLE services software block with functions to provide interfaces to make the BLE module 5041 available to the computing hardware of the sensor control device 102. These software functions can include a BLE logical interface and interface parser. BLE services offered by the communication module 5040 can include the generic access profile service, the generic attribute service, generic access service, device information service, data transmission services, and security services. The data transmission service can be a primary service used for transmitting data such as sensor control data, sensor status data, analyte measurement data (historical and current), and event log data. The sensor status data can include error data, current time active, and software state. The analyte measurement data can include information such as current and historical raw measurement values, current and historical values after processing using an appropriate algorithm or model, projections and trends of measurement levels, comparisons of other values to patient-specific averages, calls to action as determined by the algorithms or models and other similar types of data.

According to aspects of the disclosed subject matter, and as embodied herein, a sensor control device 102 can be configured to communicate with multiple devices concurrently by adapting the features of a communication protocol or medium supported by the hardware and radios of the sensor control device 102. As an example, the BLE module 5041 of the communication module 5040 can be provided with software or firmware to enable multiple concurrent connections between the sensor control device 102 as a central device and the other devices as peripheral devices, or as a peripheral device where another device is a central device.

Connections, and ensuing communication sessions, between two devices using a communication protocol such as BLE can be characterized by a similar physical channel operated between the two devices (e.g., a sensor control device 102 and data receiving device 120). The physical channel can include a single channel or a series of channels, including for example and without limitation using an agreed upon series of channels determined by a common clock and channel- or frequency-hopping sequence. Communication sessions can use a similar amount of the available communication spectrum, and multiple such communication sessions can exist in proximity. In certain embodiment, each collection of devices in a communication session uses a different physical channel or series of channels, to manage interference of devices in the same proximity.

For purpose of illustration and not limitation, reference is made to an exemplary embodiment of a procedure for a sensor-receiver connection for use with the disclosed subject matter. First, the sensor control device 102 repeatedly advertises its connection information to its environment in a search for a data receiving device 120. The sensor control device 102 can repeat advertising on a regular basis until a connection established. The data receiving device 120 detects the advertising packet and scans and filters for the sensor control device 102 to connect to through the data provided in the advertising packet. Next, data receiving device 120 sends a scan request command and the sensor control device 102 responds with a scan response packet providing additional details. Then, the data receiving device 120 sends a connection request using the Bluetooth device address associated with the data receiving device 120. The data receiving device 120 can also continuously request to establish a connection to a sensor control device 102 with a specific Bluetooth device address. Then, the devices establish an initial connection allowing them to begin to exchange data. The devices begin a process to initialize data exchange services and perform a mutual authentication procedure.

During a first connection between the sensor control device 102 and data receiving device 120, the data receiving device 120 can initialize a service, characteristic, and attribute discovery procedure. The data receiving device 120 can evaluate these features of the sensor control device 102 and store them for use during subsequent connections. Next, the devices enable a notification for a customized security service used for mutual authentication of the sensor control device 102 and data receiving device 120. The mutual authentication procedure can be automated and require no user interaction. Following the successful completion of the mutual authentication procedure, the sensor control device 102 sends a connection parameter update to request the data receiving device 120 to use connection parameter settings preferred by the sensor control device 102 and configured to maximum longevity.

The data receiving device 120 then performs sensor control procedures to backfill historical data, current data, event log, and factory data. As an example, for each type of data, the data receiving device 120 sends a request to initiate a backfill process. The request can specify a range of records defined based on, for example, the measurement value, timestamp, or similar, as appropriate. The sensor control device 102 responds with requested data until all previously unsent data in the memory of the sensor control device 102 is delivered to the data receiving device 120. The sensor control device 102 can respond to a backfill request from the data receiving device 120 that all data has already been sent. Once backfill is completed, the data receiving device 120 can notify sensor control device 102 that it is ready to receive regular measurement readings. The sensor control device 102 can send readings across multiple notifications result on a repeating basis. As embodied herein, the multiple notifications can be redundant notifications to ensure that data is transmitted correctly. Alternatively, multiple notifications can make up a single payload.

For purpose of illustration and not limitation, reference is made to an exemplary embodiment of a procedure to send a shutdown command to the sensor control device 102. The shutdown operation is executed if the sensor control device 102 is in, for example, an error state, insertion failed state, or sensor expired state. If the sensor control device 102 is not in those states, the sensor control device 102 can log the command and execute the shutdown when sensor control device 102 transitions into the error state or sensor expired state. The data receiving device 120 sends a properly formatted shutdown command to the sensor control device 102. If the sensor control device 102 is actively processing another command, the sensor control device 102 will respond with a standard error response indicating that the sensor control device 102 is busy. Otherwise, the sensor control device 102 sends a response as the command is received. Additionally, the sensor control device 102 sends a success notification through the sensor control characteristic to acknowledge the sensor control device 102 has received the command. The sensor control device 102 registers the shutdown command. At the next appropriate opportunity (e.g., depending on the current sensor state, as described herein), the sensor control device 102 will shut down.

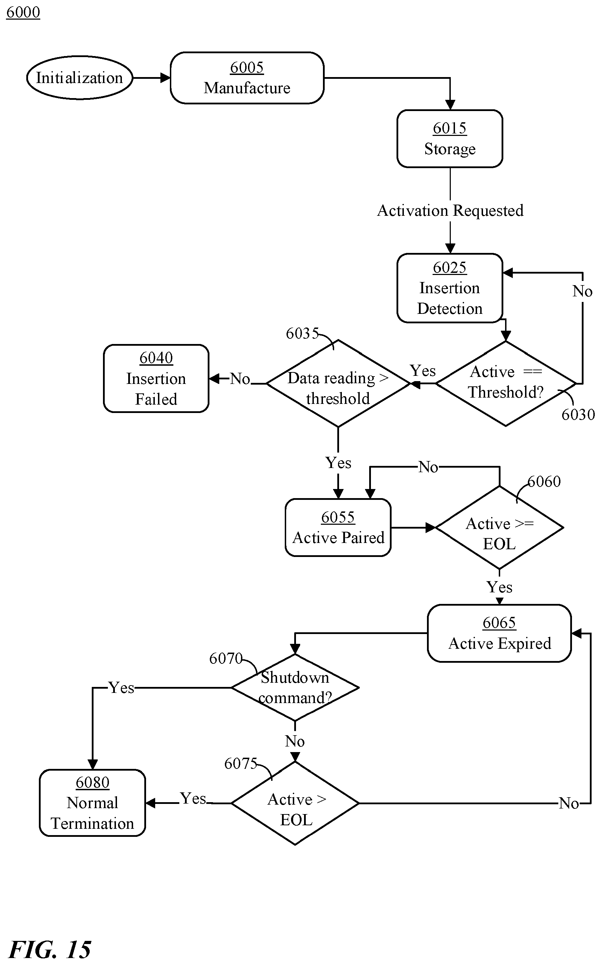

For purpose of illustration and not limitation, reference is made to the exemplary embodiment of a high-level depiction of a state machine representation 6000 of the actions that can be taken by the sensor control device 102 as shown in FIG. 15. After initialization, the sensor enters state 6005, which relates to the manufacture of the sensor control device 102. In the manufacture state 6005 the sensor control device 102 can be configured for operation, for example, the storage memory 5030 can be written. At various times while in state 6005, the sensor control device 102 checks for a received command to go to the storage state 6015. Upon entry to the storage state 6015, the sensor performs a software integrity check. While in the storage state 6015, the sensor can also receive an activation request command before advancing to the insertion detection state 6025.

Upon entry to state 6025, the sensor control device 102 can store information relating to devices authenticated to communicate with the sensor as set during activation or initialize algorithms related to conducting and interpreting measurements from the sensing hardware 5060. The sensor control device 102 can also initialize a lifecycle timer, responsible for maintaining an active count of the time of operation of the sensor control device 102 and begin communication with authenticated devices to transmit recorded data. While in the insertion detection state 6025, the sensor can enter state 6030, where the sensor control device 102 checks whether the time of operation is equal to a predetermined threshold. This time of operation threshold can correspond to a timeout function for determining whether an insertion has been successful. If the time of operation has reached the threshold, the sensor control device 102 advances to state 6035, in which the sensor control device 102 checks whether the average data reading is greater than a threshold amount corresponding to an expected data reading volume for triggering detection of a successful insertion. If the data reading volume is lower than the threshold while in state 6035, the sensor advances to state 6040, corresponding to a failed insertion. If the data reading volume satisfies the threshold, the sensor advances to the active paired state 6055.

The active paired state 6055 of the sensor control device 102 reflects the state while the sensor control device 102 is operating as normal by recording measurements, processing the measurements, and reporting them as appropriate. While in the active paired state 6055, the sensor control device 102 sends measurement results or attempts to establish a connection with a receiving device 120. The sensor control device 102 also increments the time of operation. Once the sensor control device 102 reaches a predetermined threshold time of operation (e.g., once the time of operation reaches a predetermined threshold), the sensor control device 102 transitions to the active expired state 6065. The active expired state 6065 of the sensor control device 102 reflects the state while the sensor control device 102 has operated for its maximum predetermined amount of time.

While in the active expired state 6065, the sensor control device 102 can generally perform operations relating to winding down operation and ensuring that the collected measurements have been securely transmitted to receiving devices as needed. For example, while in the active expired state 6065, the sensor control device 102 can transmit collected data and, if no connection is available, can increase efforts to discover authenticated devices nearby and establish and connection therewith. While in the active expired state 6065, the sensor control device 102 can receive a shutdown command at state 6070. If no shutdown command is received, the sensor control device 102 can also, at state 6075, check if the time of operation has exceeded a final operation threshold. The final operation threshold can be based on the battery life of the sensor control device 102. The normal termination state 6080 corresponds to the final operations of the sensor control device 102 and ultimately shutting down the sensor control device 102.

Before a sensor is activated, the ASIC 5000 resides in a low power storage mode state. The activation process can begin, for example, when an incoming RF field (e.g., NFC field) drives the voltage of the power supply to the ASIC 5000 above a reset threshold, which causes the sensor control device 102 to enter a wake-up state. While in the wake-up state, the ASIC 5000 enters an activation sequence state. The ASIC 5000 then wakes the communication module 5040. The communication module 5040 is initialized, triggering a power on self-test. The power on self-test can include the ASIC 5000 communicating with the communication module 5040 using a prescribed sequence of reading and writing data to verify the memory and one-time programmable memory are not corrupted.

When the ASIC 5000 enters the measurement mode for the first time, an insertion detection sequence is performed to verify that the sensor control device 102 has been properly installed onto the patient's body before a proper measurement can take place. First, the sensor control device 102 interprets a command to activate the measurement configuration process, causing the ASIC 5000 to enter measurement command mode. The sensor control device 102 then temporarily enters the measurement lifecycle state to run a number of consecutive measurements to test whether the insertion has been successful. The communication module 5040 or ASIC 5000 evaluates the measurement results to determine insertion success. When insertion is deemed successful, the sensor control device 102 enters a measurement state, in which the sensor control device 102 begins taking regular measurements using sensing hardware 5060. If the sensor control device 102 determines that the insertion was not successful, sensor control device 102 is triggered into an insertion failure mode, in which the ASIC 5000 is commanded back to storage mode while the communication module 5040 disables itself.

FIG. 1B further illustrates an example operating environment for providing over-the-air ("OTA") updates for use with the techniques described herein. An operator of the analyte monitoring system 100 can bundle updates for the data receiving device 120 or sensor control device 102 into updates for an application executing on the multi-purpose data receiving device 130. Using available communication channels between the data receiving device 120, the multi-purpose data receiving device 130, and the sensor control device 102, the multi-purpose data receiving device 130 can receive regular updates for the data receiving device 120 or sensor control device 102 and initiate installation of the updates on the data receiving device 120 or sensor control device 102. The multi-purpose data receiving device 130 acts as an installation or update platform for the data receiving device 120 or sensor control device 102 because the application that enables the multi-purpose data receiving device 130 to communicate with an sensor control device 102, data receiving device 120 and/or remote application server 155 can update software or firmware on a data receiving device 120 or sensor control device 102 without wide-area networking capabilities.

As embodied herein, a remote application server 155 operated by the manufacturer of the sensor control device 102 and/or the operator of the analyte monitoring system 100 can provide software and firmware updates to the devices of the analyte monitoring system 100. In particular embodiments, the remote application server 155 can provides the updated software and firmware to a user device 145 or directly to a multi-purpose data receiving device. As embodied herein, the remote application server 155 can also provide application software updates to an application storefront server 160 using interfaces provided by the application storefront. The multi-purpose data receiving device 130 can contact the application storefront server 160 periodically to download and install the updates.

After the multi-purpose data receiving device 130 downloads an application update including a firmware or software update for a data receiving device 120 or sensor control device 102, the data receiving device 120 or sensor control device 102 and multi-purpose data receiving device 130 establish a connection. The multi-purpose data receiving device 130 determines that a firmware or software update is available for the data receiving device 120 or sensor control device 102. The multi-purpose data receiving device 130 can prepare the software or firmware update for delivery to the data receiving device 120 or sensor control device 102. As an example, the multi-purpose data receiving device 130 can compress or segment the data associated with the software or firmware update, can encrypt or decrypt the firmware or software update, or can perform an integrity check of the firmware or software update. The multi-purpose data receiving device 130 sends the data for the firmware or software update to the data receiving device 120 or sensor control device 102. The multi-purpose data receiving device 130 can also send a command to the data receiving device 120 or sensor control device 102 to initiate the update. Additionally or alternatively, the multi-purpose data receiving device 130 can provide a notification to the user of the multi-purpose data receiving device 130 and include instructions for facilitating the update, such as instructions to keep the data receiving device 120 and the multi-purpose data receiving device 130 connected to a power source and in close proximity until the update is complete.

The data receiving device 120 or sensor control device 102 receives the data for the update and the command to initiate the update from the multi-purpose data receiving device 130. The data receiving device 120 can then install the firmware or software update. To install the update, the data receiving device 120 or sensor control device 102 can place or restart itself in a so-called "safe" mode with limited operational capabilities. Once the update is completed, the data receiving device 120 or sensor control device 102 re-enters or resets into a standard operational mode. The data receiving device 120 or sensor control device 102 can perform one or more self-tests to determine that the firmware or software update was installed successfully. The multi-purpose data receiving device 130 can receive the notification of the successful update. The multi-purpose data receiving device 130 can then report a confirmation of the successful update to the remote application server 155.

In particular embodiments, the storage memory 5030 of the sensor control device 102 includes one-time programmable (OTP) memory. The term OTP memory can refer to memory that includes access restrictions and security to facilitate writing to particular addresses or segments in the memory a predetermined number of times. The memory 5030 can be prearranged into multiple pre-allocated memory blocks or containers. The containers are pre-allocated into a fixed size. If storage memory 5030 is one-time programming memory, the containers can be considered to be in a non-programmable state. Additional containers which have not yet been written to can be placed into a programmable or writable state. Containerizing the storage memory 5030 in this fashion can improve the transportability of code and data to be written to the storage memory 5030. Updating the software of a device (e.g., the sensor device described herein) stored in an OTP memory can be performed by superseding only the code in a particular previously-written container or containers with updated code written to a new container or containers, rather than replacing the entire code in the memory. In a second embodiment, the memory is not prearranged. Instead, the space allocated for data is dynamically allocated or determined as needed. Incremental updates can be issued, as containers of varying sizes can be defined where updates are anticipated.

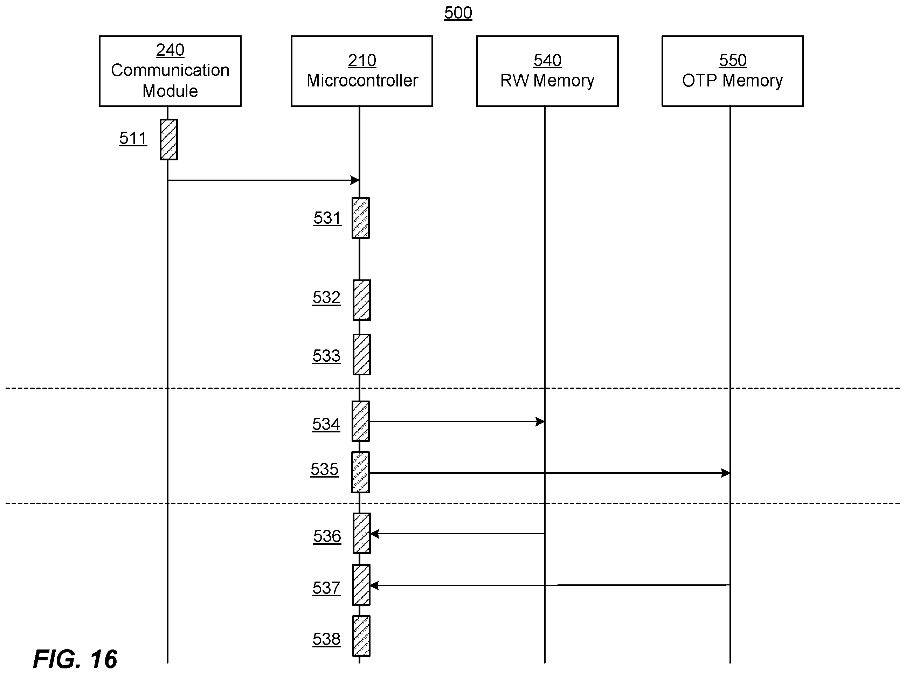

FIG. 16 is a diagram illustrating an example operational and data flow for over-the-air (OTA) programming of a storage memory 5030 in a sensor control device 102 as well as use of the memory after the OTA programming in execution of processes by the sensor device 110 according to the disclosed subject matter. In the example OTA programming 500 illustrated in FIG. 5, a request is sent from an external device (e.g., the data receiving device 130) to initiate OTA programming (or re-programming). At 511, a communication module 5040 of a sensor device 110 receives an OTA programming command. The communication module 5040 sends the OTA programming command to the microcontroller 5010 of the sensor device 110.

At 531, after receiving the OTA programming command, the microcontroller 5010 validates the OTA programming command. The microcontroller 5010 can determine, for example, whether the OTA programming command is signed with an appropriate digital signature token. Upon determining that the OTA programming command is valid, the microcontroller 5010 can set the sensor device into an OTA programming mode. At 532, the microcontroller 5010 can validate the OTA programming data. At 533, The microcontroller 5010 can reset the sensor device 110 to re-initialize the sensor device 110 in a programming state. Once the sensor device 110 has transitioned into the OTA programming state, the microcontroller 5010 can begin to write data to the rewriteable memory 540 (e.g., memory 5020) of the sensor device at 534 and write data to the OTP memory 550 of the sensor device at 535 (e.g., storage memory 5030). The data written by the microcontroller 5010 can be based on the validated OTA programming data. The microcontroller 5010 can write data to cause one or more programming blocks or regions of the OTP memory 550 to be marked invalid or inaccessible. The data written to the free or unused portion of the OTP memory can be used to replace invalidated or inaccessible programming blocks of the OTP memory 550. After the microcontroller 5010 writes the data to the respective memories at 534 and 535, the microcontroller 5010 can perform one or more software integrity checks to ensure that errors were not introduced into the programming blocks during the writing process. Once the microcontroller 5010 is able to determine that the data has been written without errors, the microcontroller 5010 can resume standard operations of the sensor device.

In execution mode, at 536, the microcontroller 5010 can retrieve a programming manifest or profile from the rewriteable memory 540. The programming manifest or profile can include a listing of the valid software programming blocks and can include a guide to program execution for the sensor control device 102. By following the programming manifest or profile, the microcontroller 5010 can determine which memory blocks of the OTP memory 550 are appropriate to execute and avoid execution of out-of-date or invalidated programming blocks or reference to out-of-date data. At 537, the microcontroller 5010 can selectively retrieve memory blocks from the OTP memory 550. At 538, the microcontroller 5010 can use the retrieved memory blocks, by executing programming code stored or using variable stored in the memory.

As embodied herein a first layer of security for communications between the sensor control device 102 and other devices can be established based on security protocols specified by and integrated in the communication protocols used for the communication. Another layer of security can be based on communication protocols that necessitate close proximity of communicating devices. Furthermore certain packets and/or certain data included within packets can be encrypted while other packets and/or data within packets is otherwise encrypted or not encrypted. Additionally or alternatively, application layer encryption can be used with one or more block ciphers or stream ciphers to establish mutual authentication and communication encryption with other devices in the analyte monitoring system 100.

The ASIC 5000 of the sensor control device 102 can be configured to dynamically generate authentication and encryption keys using data retained within the storage memory 5030. The storage memory 5030 can also be pre-programmed with a set of valid authentication and encryption keys to use with particular classes of devices. The ASIC 5000 can be further configured to perform authentication procedures with other devices using received data and apply the generated key to sensitive data prior to transmitting the sensitive data. The generated key can be unique to the sensor control device 102, unique to a pair of devices, unique to a communication session between an sensor control device 102 and other device, unique to a message sent during a communication session, or unique to a block of data contained within a message.

Both the sensor control device 102 and a data receiving device 120 can ensure the authorization of the other party in a communication session to, for example, issue a command or receive data. In particular embodiments, identity authentication can be performed through two features. First, the party asserting its identity provides a validated certificate signed by the manufacturer of the device or the operator of the analyte monitoring system 100. Second, authentication can be enforced through the use of public keys and private keys, and shared secrets derived therefrom, established by the devices of the analyte monitoring system 100 or established by the operator of the analyte monitoring system 100. To confirm the identity of the other party, the party can provide proof that the party has control of its private key.

The manufacturer of the sensor control device 102, data receiving device 120, or provider of the application for multi-purpose data receiving device 130 can provide information and programming necessary for the devices to securely communicate through secured programming and updates. For example, the manufacturer can provide information that can be used to generate encryption keys for each device, including secured root keys for the sensor control device 102 and optionally for the data receiving device 120 that can be used in combination with device-specific information and operational data (e.g., entropy-based random values) to generate encryption values unique to the device, session, or data transmission as need.

Analyte data associated with a user is sensitive data at least in part because this information can be used for a variety of purposes, including for health monitoring and medication dosing decisions. In addition to user data, the analyte monitoring system 100 can enforce security hardening against efforts by outside parties to reverse-engineering. Communication connections can be encrypted using a device-unique or session-unique encryption key. Encrypted communications or unencrypted communications between any two devices can be verified with transmission integrity checks built into the communications. Sensor control device 102 operations can be protected from tampering by restricting access to read and write functions to the memory 5020 via a communication interface. The sensor can be configured to grant access only to known or "trusted" devices, provided in a "whitelist" or only to devices that can provide a predetermined code associated with the manufacturer or an otherwise authenticated user. A whitelist can represent an exclusive range, meaning that no connection identifiers besides those included in the whitelist will be used, or a preferred range, in which the whitelist is searched first, but other devices can still be used. The sensor control device 102 can further deny and shut down connection requests if the requestor cannot complete a login procedure over a communication interface within a predetermined period of time (e.g., within four seconds). These characteristics safeguard against specific denial of service attacks, and in particular against denial of service attacks on a BLE interface.

As embodied herein, the analyte monitoring system 100 can employ periodic key rotation to further reduce the likelihood of key compromise and exploitation. A key rotation strategy employed by the analyte monitoring system 100 can be designed to support backward compatibility of field-deployed or distributed devices. As an example, the analyte monitoring system 100 can employ keys for downstream devices (e.g., devices that are in the field or cannot be feasibly provided updates) that are designed to be compatible with multiple generations of keys used by upstream devices.

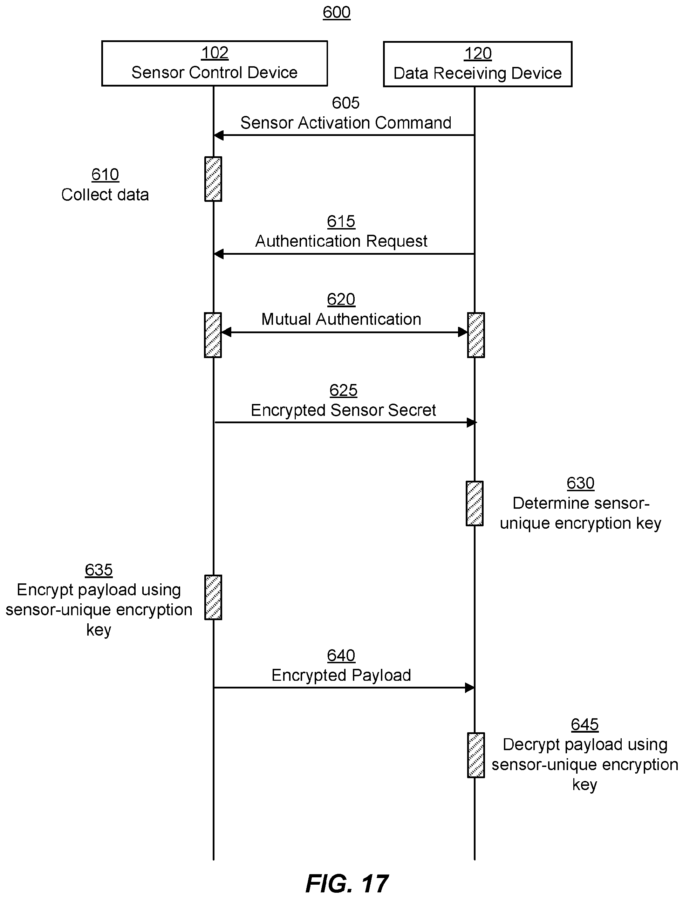

For purpose of illustration and not limitation, reference is made to the exemplary embodiment of a message sequence diagram 600 for use with the disclosed subject matter as shown in FIG. 17 and demonstrating an example exchange of data between a pair of devices, particularly a sensor control device 102 and a data receiving device 120. The data receiving device 120 can, as embodied herein, be a data receiving device 120 or a multi-purpose data receiving device 130. At step 605, the data receiving device 120 can transmit a sensor activation command 605 to the sensor control device 102, for example via a short-range communication protocol. The sensor control device 102 can, prior to step 605 be in a primarily dormant state, preserving its battery until full activation is needed. After activation during step 610, the sensor control device 102 can collect data or perform other operations as appropriate to the sensing hardware 5060 of the sensor control device 102. At step 615 the data receiving device 120 can initiate an authentication request command 615. In response to the authentication request command 615, both the sensor control device 102 and data receiving device 120 can engage in a mutual authentication process 620. The mutual authentication process 620 can involve the transfer of data, including challenge parameters that allow the sensor control device 102 and data receiving device 120 to ensure that the other device is sufficiently capable of adhering to an agreed-upon security framework described herein. Mutual authentication can be based on mechanisms for authentication of two or more entities to each other with or without on-line trusted third parties to verify establishment of a secret key via challenge-response. Mutual authentication can be performed using two-, three-, four-, or five-pass authentication, or similar versions thereof.

Following a successful mutual authentication process 620, at step 625 the sensor control device 102 can provide the data receiving device 120 with a sensor secret 625. The sensor secret can contain sensor-unique values and be derived from random values generated during manufacture. The sensor secret can be encrypted prior to or during transmission to prevent third-parties from accessing the secret. The sensor secret 625 can be encrypted via one or more of the keys generated by or in response to the mutual authentication process 620. At step 630, the data receiving device 120 can derive a sensor-unique encryption key from the sensor secret. The sensor-unique encryption key can further be session-unique. As such, the sensor-unique encryption key can be determined by each device without being transmitted between the sensor control device 102 or data receiving device 120. At step 635, the sensor control device 102 can encrypt data to be included in payload. At step 640, the sensor control device 102 can transmit the encrypted payload 640 to the data receiving device 120 using the communication link established between the appropriate communication models of the sensor control device 102 and data receiving device 120. At step 645, the data receiving device 120 can decrypt the payload using the sensor-unique encryption key derived during step 630. Following step 645, the sensor control device 102 can deliver additional (including newly collected) data and the data receiving device 120 can process the received data appropriately.

As discussed herein, the sensor control device 102 can be a device with restricted processing power, battery supply, and storage. The encryption techniques used by the sensor control device 102 (e.g., the cipher algorithm or the choice of implementation of the algorithm) can be selected based at least in part on these restrictions. The data receiving device 120 can be a more powerful device with fewer restrictions of this nature. Therefore, the data receiving device 120 can employ more sophisticated, computationally intense encryption techniques, such as cipher algorithms and implementations.

The sensor control device 102 can be configured to alter its discoverability behavior to attempt to increase the probability of the receiving device receiving an appropriate data packet and/or provide an acknowledgement signal or otherwise reduce restrictions that can be causing an inability to receive an acknowledgement signal. Altering the discoverability behavior of the sensor control device 102 can include, for example and without limitation, altering the frequency at which connection data is included in a data packet, altering how frequently data packets are transmitted generally, lengthening or shortening the broadcast window for data packets, altering the amount of time that the sensor control device 102 listens for acknowledgement or scan signals after broadcasting, including directed transmissions to one or more devices (e.g., through one or more attempted transmissions) that have previously communicated with the sensor control device 102 and/or to one or more devices on a whitelist, altering a transmission power associated with the communication module when broadcasting the data packets (e.g., to increase the range of the broadcast or decrease energy consumed and extend the life of the battery of the analyte sensor), altering the rate of preparing and broadcasting data packets, or a combination of one or more other alterations. Additionally, or alternatively, the receiving device can similarly adjust parameters relating to the listening behavior of the device to increase the likelihood of receiving a data packet including connection data.

As embodied herein, the sensor control device 102 can be configured to broadcast data packets using two types of windows. The first window refers to the rate at which the sensor control device 102 is configured to operate the communication hardware. The second window refers to the rate at which the sensor control device 102 is configured to be actively transmitting data packets (e.g., broadcasting). As an example, the first window can indicate that the sensor control device 102 operates the communication hardware to send and/or receive data packets (including connection data) during the first 2 seconds of each 60 second period. The second window can indicate that, during each 2 second window, the sensor control device 102 transmits a data packet every 60 milliseconds. The rest of the time during the 2 second window, the sensor control device 102 is scanning. The sensor control device 102 can lengthen or shorten either window to modify the discoverability behavior of the sensor control device 102.

In particular embodiments, the discoverability behavior of the analyte sensor can be stored in a discoverability profile, and alterations can be made based on one or more factors, such as the status of the sensor control device 102 and/or by applying rules based on the status of the sensor control device 102. For example, when the battery level of the sensor control device 102 is below a certain amount, the rules can cause the sensor control device 102 to decrease the power consumed by the broadcast process. As another example, configuration settings associated with broadcasting or otherwise transmitting packets can be adjusted based on the ambient temperature, the temperature of the sensor control device 102, or the temperature of certain components of communication hardware of the sensor control device 102. In addition to modifying the transmission power, other parameters associated with the transmission capabilities or processes of the communication hardware of the sensor control device 102 can be modified, including, but not limited to, transmission rate, frequency, and timing. As another example, when the analyte data indicates that the subject is, or is about to be, experiencing a negative health event, the rules can cause the sensor control device 102 to increase its discoverability to alert the receiving device of the negative health event.

As embodied herein, certain calibration features for the sensing hardware 5060 of the sensor control device 102 can be adjusted based on external or interval environment features as well as to compensate for the decay of the sensing hardware 5060 during expended period of disuse (e.g., a "shelf time" prior to use). The calibration features of the sensing hardware 5060 can be autonomously adjusted by the sensor control device 102 (e.g., by operation of the ASIC 5000 to modify features in the memory 5020 or storage 5030) or can be adjusted by other devices of the analyte monitoring system 100.

As an example, sensor sensitivity of the sensing hardware 5060 can be adjusted based on external temperature data or the time since manufacture. When external temperatures are monitored during the storage of the sensors, the disclosed subject matter can adaptively change the compensation to sensor sensitivity over time when the device experiences changing storage conditions. According to the invention, adaptive sensitivity adjustment can be performed in an "active" storage mode where the sensor control device 102 wakes up periodically to measure temperature. These features can save the battery of the analyte device and extend the lifespan of the analyte sensors. At each temperature measurement, the sensor control device 102 can calculate a sensitivity adjustment for that time period based on the measured temperature. Then, the temperature-weighted adjustments can be accumulated over the active storage mode period to calculate a total sensor sensitivity adjustment value at the end of the active storage mode (e.g., at insertion). Similarly, at insertion, the sensor control device 102 can determine the time difference between manufacture of the sensor control device 102 (which can be written to the storage 5030 of the ASIC 5000) or the sensing hardware 5060 and modify sensor sensitivity or other calibration features according to one or more known decay rates or formulas.

Additionally, for purpose of illustration and not limitation, as embodied herein, sensor sensitivity adjustments can account for other sensor conditions, such as sensor drift. Sensor sensitivity adjustments can be hardcoded into the sensor control device 102 during manufacture, for example in the case of sensor drift, based on an estimate of how much an average sensor can drift. Sensor control device 102 can use a calibration function that has time-varying functions for sensor offset and gain, which can account for drift over a wear period of the sensor. Thus, sensor control device 102 can utilize a function used to transform an interstitial current to interstitial glucose utilizing device-dependent functions describing sensor control device 102 drift over time, and which can represent sensor sensitivity, and can be device specific, combined with a baseline of the glucose profile. Such functions to account for sensor sensitivity and drift can improve sensor control device 102 accuracy over a wear period and without involving user calibration.

The sensor control device 102 detects raw measurement values from sensing hardware 5060. On-sensor processing can be performed, such as by one or more models trained to interpret the raw measurement values. Models can be machine learned models trained off-device to detect, predict, or interpret the raw measurement values to detect, predict, or interpret the levels of one or more analytes. Additional trained models can operate on the output of the machine learning models trained to interact with raw measurement values. As an example, models can be used to detect, predict, or recommend events based on the raw measurements and type of analyte(s) detected by the sensing hardware 5060. Events can include, initiation or completion of physical activity, meals, application of medical treatment or medication, emergent health events, and other events of a similar nature.

Models can be provided to the sensor control device 102, data receiving device 120, or multi-purpose data receiving device 130 during manufacture or during firmware or software updates. Models can be periodically refined, such as by the manufacturer of the sensor control device 102 or the operator of the analyte monitoring system 100, based on data received from the sensor control device 102 and data receiving devices of an individual user or multiple users collectively. In certain embodiments, the sensor control device 102 includes sufficient computational components to assist with further training or refinement of the machine learned models, such as based on unique features of the user to which the sensor control device 102 is attached. Machine learning models can include, by way of example and not limitation, models trained using or encompassing decision tree analysis, gradient boosting, ada boosting, artificial neural networks or variants thereof, linear discriminant analysis, nearest neighbor analysis, support vector machines, supervised or unsupervised classification, and others. The models can also include algorithmic or rules-based models in addition to machine learned models. Model-based processing can be performed by other devices, including the data receiving device 120 or multi-purpose data receiving device 130, upon receiving data from the sensor control device 102 (or other downstream devices).

Data transmitted between the sensor control device 102 and a data receiving device 120 can include raw or processed measurement values. Data transmitted between the sensor control device 102 and data receiving device 120 can further include alarms or notification for display to a user. The data receiving device 120 can display or otherwise convey notifications to the user based on the raw or processed measurement values or can display alarms when received from the sensor control device 102. Alarms that can be triggered for display to the user include alarms based on direct analyte values (e.g., one-time reading exceeding a threshold or failing to satisfy a threshold), analyte value trends (e.g., average reading over a set period of time exceeding a threshold or failing to satisfy a threshold; slope); analyte value predictions (e.g., algorithmic calculation based on analyte values exceeds a threshold or fails to satisfy a threshold), sensor alerts (e.g., suspected malfunction detected), communication alerts (e.g., no communication between sensor control device 102 and data receiving device 120 for a threshold period of time; unknown device attempting or failing to initiate a communication session with the sensor control device 102), reminders (e.g., reminder to charge data receiving device 120; reminder to take a medication or perform other activity), and other alerts of a similar nature. For purpose of illustration and not limitation, as embodied herein, the alarm parameters described herein can be configurable by a user or can be fixed during manufacture, or combinations of user-settable and non-user-settable parameters.

As described herein, a software library integrated within software executing on a receiving device can facilitate communicating with analyte sensors and permitting third party applications access to the sensor data for use in medically necessary applications or applications related to the well-being of the user. The software library can be implemented independently of the sensors and integrated within third party applications to allow access to the sensor data. A sensor control module can further communicate with the sensor assemblies in such a manner to receive data simultaneously or substantially simultaneously from a plurality of such sensor assemblies. The system further enables the transfer of sensor information from the sensor control module to a remote management module.

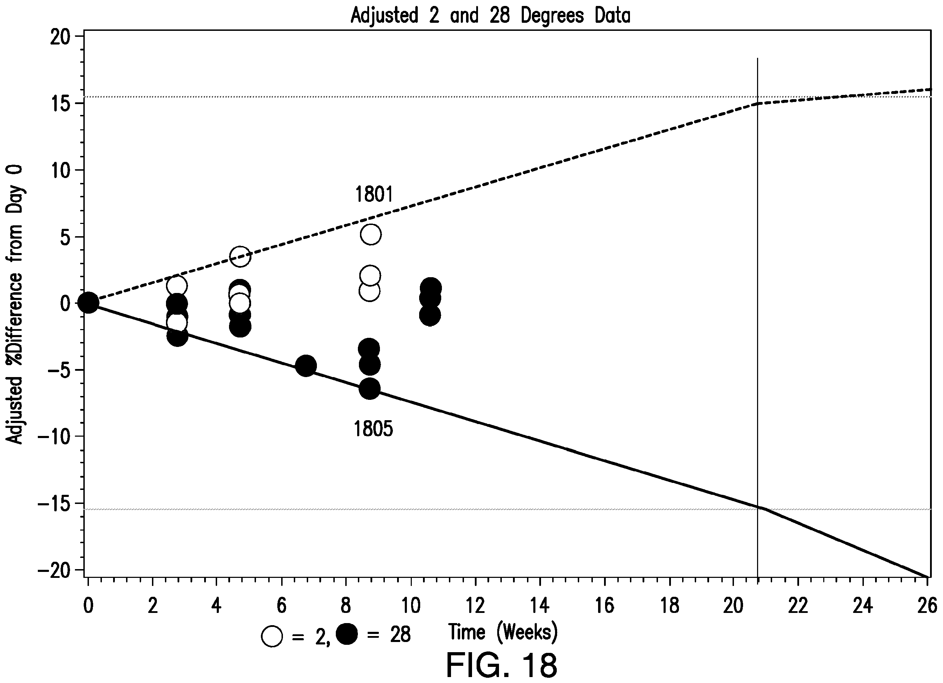

For purpose of illustration and not limitation, reference is now made to FIG. 18. FIG. 18 illustrates an example that external temperature changes of the sensors may affect the sensitivity of sensors. For purpose of illustration, line 1801 represents a sensitivity change of sensors over time when their external temperature is 2 °C. Line 1805 represents a sensitivity change of sensors over time when their external temperature is 28 °C. To meet an acceptable window of +/- 15% change to the sensor sensitivity, if a fixed slope compensation is applied to the sensor sensitivity, the sensor expiry would be limited to about 21 weeks from the sensor pack-out date.

In some examples, there may be manufacturing margin for the sensor sensitivity, and the sensor expiry may be shortened to about 18 weeks. In other examples, such as where sensors are stored in refrigerators, the loss of sensitivity can be compensated by a fixed slope calculation as much as about 13% with an expiry of about 18 weeks. The lifespan of sensors can be extended using systems and methods to perform sensitivity adjustment according to the disclosed subject matter herein.

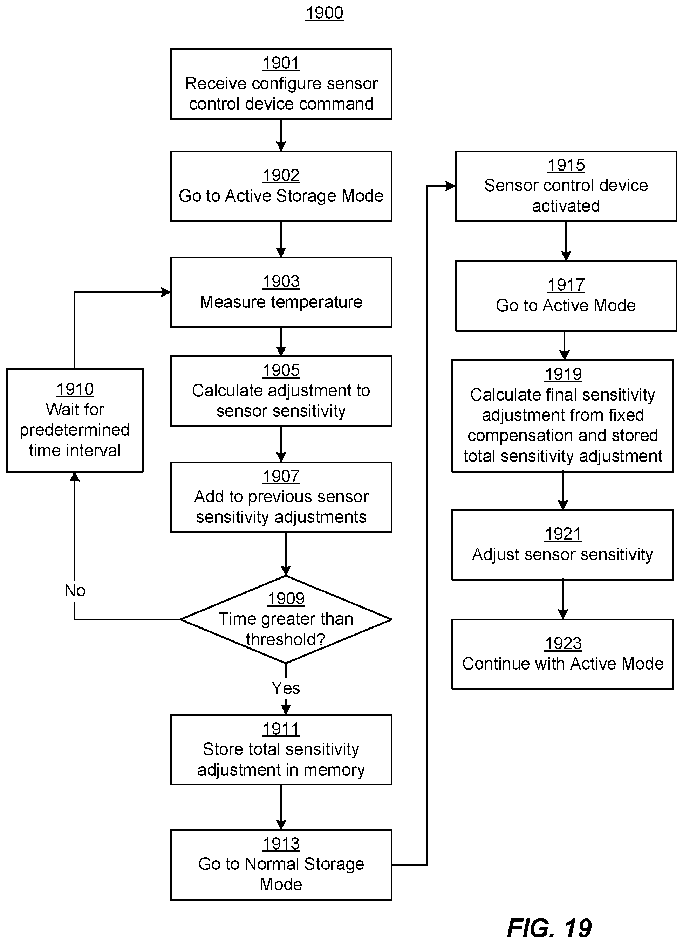

For purpose of illustration and not limitation, reference is made to an exemplary process 1900 to adjust the sensor sensitivity of a sensor control device 102 (also referred to herein as a "patch") based on temperature data according to the disclosed subject matter as shown in FIG. 19. Although described herein a process running on the sensor control device 102, process 1900 of FIG. 19 can be performed on any devices of the analyte monitoring system 100, including data receiving device 120, multi-purpose data receiving device 130, user device 145, or remote server 155, or any device in communication with the analyte monitoring system 100. Process 1900 of FIG. 19 can be implemented as software, hardware, firmware or any other programming modality.

For example, and as embodied herein, when external temperatures are monitored during the storage of the sensors or sensor control devices 102, the disclosed subject matter can adaptively change compensation applied to sensor sensitivity over time when the sensor control device 102 experiences changing storage conditions. For purpose of illustration not limitation, the adaptive sensitivity adjustment of process 1900 can be performed in an Active Storage mode where the sensor control device 102 ASIC 5000 and communication module 5040 (e.g., the BLE radio 5041) are put into reduced power modes, and can wake up periodically to measure temperature. These features can save the battery of the medical device and extend the lifespan of the medical sensors.

At each temperature measurement, the sensor control device 102 can calculate a sensitivity adjustment for that time period based on the measured temperature. Then, the temperature-weighted adjustments can be accumulated over the Active Storage mode period to calculate a total sensor sensitivity adjustment value at the end of the Active Storage mode. The length of the Active Storage mode is limited by the battery capacity of the device. To extend a shelf life of a sensor control device 102 (including the incorporated sensor), process 1900 can cause the sensor control device 102 to revert to a standard or normal storage mode at the end of maximum Active Storage mode. During a second phase of storage, a fixed sensitivity adjustment can be applied by process 1900 to the sensor control device 102. The fixed compensation can be calculated when the sensor control device 102 is activated (e.g., by a user before, during, or after insertion), and can be added to the previously-stored total adaptive adjustment in the Active Storage mode as a final sensitivity adjustment.

As embodied herein, process 1900 can begin in or be performed in a Pack Line NFC station. The Pack Line NFC station configures the sensor control device 102. Portions of the process 1900 can extend to the time when a user activates the sensor control device 102. Referring to FIG. 19, the process can begin when the Pack Line NFC station sends a command to the sensor control device 102 to configured the sensor control device 102 (1901). When the sensor control device 102 is in storage, the sensor control device 102 may be caused to enter an Active Storage mode for the sensor (1902). For example, a user can cause the sensor control device 102 to enter the Active Storage mode, or the sensor control device 102 can enter the Active Storage mode automatically.

When sensor control device 102 is in the Active Storage mode, at least one processor for sensor control device 102 can cause a temperature sensor (or a thermistor) to measure an external temperature of the sensor (1903) corresponding to a first measurement time. After the external temperature of the first measurement time is obtained, the processor is configured to calculate an adjustment to the sensor sensitivity $S_{ADJn}$ corresponding to the measurement time (1905).

For purpose of illustration and not limitation, $S_{ADJn} = M \cdot T_n \cdot \Delta t$, wherein M can be a sensor sensitivity change rate, wherein $T_n$ is the measured external temperature for the sensor corresponding to the measurement time, and where $\Delta t$ is a time interval between a first measurement time and a second measurement time. As embodied herein, $S_{ADJ1} = M \cdot T_1 \cdot \Delta t$, wherein $\Delta t$ can be 1 hour, and $T_1$ is the measured external temperature corresponding to the first time.

As embodied herein, $S_{ADJN}$ is a total sensitivity adjustment. $S_{ADJN}$ can be calculated as a sum of sensitivity adjustments based on a plurality of measured external temperatures. The process of the sensor control device 102 can add the calculated sensitivity adjustment to a running total of previous sensor sensitivity adjustments (1907). For purpose of illustration and not limitation, $S_{ADJN} = \sum_{n=1}^{n=N} M \cdot T_n \cdot \Delta t$, wherein M can be the sensor control device 102 sensitivity change rate, wherein $T_n$ is a measured external temperature for the sensor corresponding to a measurement time $t_n$, where $\Delta t$ is a time interval between the closest temperature measurements, and where $T_N$ can be a last time for temperature measurement.

As embodied herein, additionally or alternatively, when the external temperature is below or equal to 0 °C, the sensor sensitivity change rate M is 0, and a total sensitivity adjustment is 0. When the external temperature is equal to or above a maximum temperature $T_{max}$ for which a sensitivity adjustment is calculated, the sensor sensitivity change rate M is $M_{Tmax}$, where $M_{Tmax}$ is the sensor sensitivity change rate at the external temperature $T_{max}$. $T_{max}$ can be any suitable temperatures for the corresponding sensor control devices 102. For purpose of illustration not limitation, M can be $\frac{M_{Tmax}}{T_{max}}$ (in a unit of % / hour/ °C). As embodied herein, $T_{max}$ can be 30 °C, $M_{Tmax}$ can be 0.003 %/hour, and M can be 0.001 %/hour/°C. For purpose of illustration only, the time interval between each two measurements can be 1 hour. There is no need to store each temperature measurement during the Active Storage mode.

As embodied herein, after the total sensitivity adjustment is calculated, the processor of sensor control device 102 can determine whether the measured time is less than a predetermined time threshold $t_{max}$ or otherwise within a predefined range of valid times (1909). If the measured time is within the predetermined time threshold, the one or more processor is configured to measure a next external temperature after a waiting for a predetermined time interval $\Delta t$ (1910) and to calculate a sensitivity adjustment based on the next temperature measurement. If the measured time is equal to or above the predetermined time threshold, the total sensitivity adjustment can be stored in one or more memories of the device (1911). For purpose of illustration not limitation, one of the memories can be a One Time Programmable (OTP) memory. The stored total sensitivity adjustment can be used by sensor control device 102 to determine a final sensitivity adjustment in the Active mode. Because there is no need to store every external temperature measurement in the one or more memories, battery power of the sensor control device 102 can be saved.

As embodied herein, after the total sensitivity adjustment $S_{ADJN}$ is stored in the memory, the sensor control device 102 can enter a Normal Storage Mode (1913) without continuing to measure the external temperatures. When the sensor control device 102 is later activated (1915), the process can cause sensor control device 102 to enter an Active Mode (1917). In the Active Mode, the sensor control device 102 can calculate a final sensitivity adjustment to the sensor. For purpose of illustration not limitation, the final sensitivity adjustment $S_{ADJ}$ can be calculated from the stored total sensitivity adjustment and a fixed compensation to the sensor sensitivity (1919). As an example, the final sensitivity adjustment can be a weighted or non-weighted sum of the stored total sensitivity adjustment and the fixed compensation. As embodied herein, the fixed compensation can be calculated by multiplying the maximum temperature $T_{max}$, by a time interval between the Active Storage mode and the Active mode, by a fixed sensor sensitivity change rate $M_f$. For purpose of illustration not limitation, $M_f$ can be a sensor sensitivity change rate M corresponding to a half of the maximum temperature $T_{max}$. For purpose of illustration only, $M_f$ can be a sensitivity change rate M corresponding to 15 °C.

As embodied herein, during operation sensor control device 102 can adjust the sensor sensitivity (1921). As an example and not limitation, the sensor control device 102 can adjust the sensor sensitivity by multiplying the sensor sensitivity by a difference between 100 and the final sensitivity adjustment divided by 100. The device with an adjusted sensor sensitivity can continue to work in an Active mode (1923).

Additionally or alternatively, during the Active Storage mode, sensor control device 102 can be caused to reset. If the ASIC 5000 according to the disclosed subject matter above goes through a non-recoverable reset or if the communication module 5040 (e.g., the BLE module 5041) goes through a reset, sensor control device 102 can terminate the adaptive adjustment process. This is at least in part because temperature measurements can be difficult to acquire if the ASIC is powered down, and any loss of adaptive adjustment time will result in some level of error for the sensitivity adjustment. If the ASIC 5000 goes through a recoverable reset, such as from an electro-static discharge (ESD), sensor control device 102 can handle this as it does in the Active mode and continue in the Active Storage mode. Sensor control device 102 can reconfigure the ASIC shadow registers to disable the front-end circuits to enter a reduced power mode.

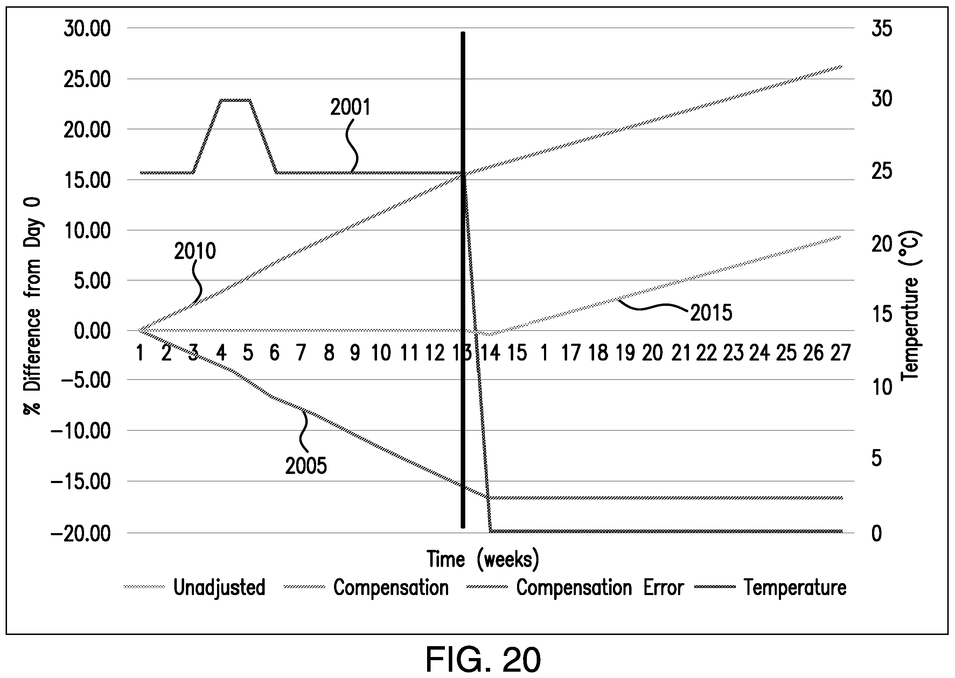

For purpose of illustration and not limitation, reference is made to FIG. 20 as an exemplary diagram illustrating the adaptive sensitivity adjustment and the fixed compensation of the sensor sensitivity according to the disclosed subject matter. The diagram in Fig. 20 illustrates the sensitivity change and adjustment when the external temperature mostly stays at around 25 °C. The X-axis represents the time since sensor control device 102 is in storage, with units in weeks. The left vertical axis represents a sensitivity difference percentage from an original sensitivity corresponding to the sensor when it is first in storage (Day 0). The right vertical axis represents an external temperature, with units in °C. For purpose of illustration, the adaptive sensitivity adjustment according to the disclosed subject matter can last for around 13 weeks ($t_{max}$ = 13 weeks). As embodied herein, after 13 weeks, a fixed sensitivity change rate $M_f$ is shown for the sensitivity change rate at around a temperature of 15°C.

For purpose of illustration not limitation, line 2001 represents the changes of external temperature of the sensor in storage. As illustrated by line 2001, from week 1 to week 3, the external temperature is around 25 °C. From week 3 to week 4, the external temperature rises from around 25 °C to around 30 °C. Form week 4 to week 5, the external temperature stays around 23 °C. From week 5 to week 6, the external temperature decreases from around 30 °C to around 25 °C. From week 6 to week 13, the external temperature stays around 15 °C. From week 13 to week 14, the external temperature decreases from around 25 °C to around 0 °C. From week 14 to week 27, the external temperature stays around 0 °C.

For purpose of illustration not limitation, line 2005 represents the changes of sensor sensitivity without sensitivity adjustment due to the changes of external temperature. From week 1 to around week 13, the sensor decreases around 15% in its sensitivity. From week 13 to week 14, the sensor decreases from 15% to around 17% in its sensitivity. From week 14 to week 27, the sensor sensitivity remains around 83% of its original sensitivity from Day 0.

For purpose of illustration not limitation, line 2010 represents the calculated sensitivity adjustment to the sensor. From Week 1 to Week 13, an adaptive adjustment in the Active Storage mode according to the disclosed subject matter is performed and calculated. The adaptive adjustment from Week 1 to Week 13 can be as much as about 15%. For week 13 to Week 27, a fixed compensation in the Active mode according to the disclosed subject matter is performed and calculated. The fixed compensation from Week 13 to Week 27 can be as much as about 9.3%. Combining the fixed compensation and the stored adaptive adjustment together, the final sensitivity adjustment can be as much as around 24.3%.

For purpose of illustration not limitation, line 2015 represents the compensation error to the sensor after considering the calculated adjustment. From Week 1 to Week 13, after the calculation of the adaptive sensitivity adjustment, the compensation error is about 0. From Week 13 to Week 14, the compensation error to the sensitivity using a fixed compensation is about 1 %. Form Week 14 to Week 27, the compensation error to the sensitivity using a fixed compensation is as much as 9.3%, which is still well within a 15% sensitivity change threshold. As illustrated by the diagram in FIG. 20, the sensor expiry can be limited to, at least, about 27 weeks, with the sensitivity adjustment performed according to the disclosed subject matter.

During the performance of the sensitivity adjustment according to the disclosed subject matter, there are a few factors that may affect the accuracy of the sensor sensitivity adjustment. For purpose of illustration only, such factors can include sensor variation, temperature measurement accuracy, and timing error.

One factor that can affect the accuracy of adaptive sensor sensitivity adjustment is the sensor sensitivity change rate M across different sensor lots. For purpose of illustration not limitation, with current obtained data, M is anticipated to vary by as much as a window of +/- 36%. For a period of 12 weeks, this can amount to as much as +/- 3.7% error in sensor sensitivity adjustment. As such, to increase the accuracy of the sensitivity adjustment, an accurate sensitivity change rate M should correspond to specific sensors.

Additionally, sometimes the real time aging of multiple sensor lots tested across multiple temperatures can lead to nonlinear changes of the sensitivity. However, current sensitivity analysis of deviation from linear for this function has shown that it is quite tolerant of nonlinearity. Thus, the nonlinear changes can be a neglectable source of error.

Another factor that can affect the accuracy of adaptive sensor sensitivity adjustment is the accuracy of temperature measurement. Sensor control device 102 can include a thermistor used as the temperature sensor. The thermistor can achieve +/- 0.5 °C accuracy at sensor control device 102 mount surface. During storage, there can be a negligible thermal gradient between the thermistor (the temperature sensor) and sensor control device 102 itself, at least in part because sensor control device 102 can be contained within an Applicator which is enclosed in the carton, which can prevent or inhibit air movement across sensor control device 102. Furthermore, storage conditions generally change very slowly, such as variations between night and daytime. Moreover, the temperature characterization can be done with Applicators in cartons as well. Thus, the temperature measurement can be a negligible source of error.

Another factor that can affect the accuracy of adaptive sensor sensitivity adjustment is the accuracy of temperature measurement. During the Active Storage mode, sensor control device 102 can keep time based on a low power Resistance-Capacitance (RC) oscillator in the communication module 5040. When in a free-running mode, the oscillator has an accuracy of 10%. However, when a temperature measurement is made, sensor control device 102 can cause the RC oscillator to be calibrated to a 500 PPM high frequency crystal oscillator in the chip. Effectively, the time base for sensor control device 102 in Active Storage mode will be accurate to within 500 PPM, which can have a timing error of up to 1 hour after 12 months if the crystal is at the edge of its accuracy specification. The timing error can be considered a negligible source of error.

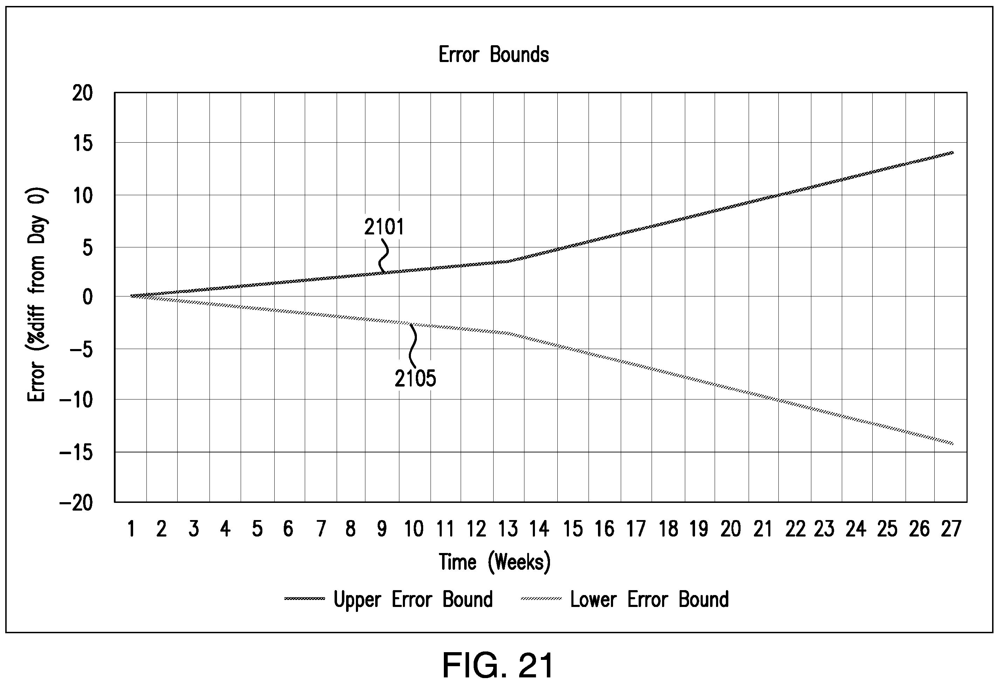

Combining the sensor variation error and the error during the fixed compensation period in the Active mode, the overall error after 27 weeks from day 0 of the sensor is up to +/- 14.2%, based on the worst case of each error. Reference will now be made to FIG. 21 to illustrate the upper and lower maximum overall error bounds. In FIG. 21, X-axis represents the time since sensor control device 102 is in storage, with units in weeks. Y-axis represents the sensitivity error bound using sensor control device 102 in the Active Storage mode and Active mode.

For purpose of illustration not limitation, line 2101 represents an upper error bound for sensor control device 102, and line 2105 represents a lower error bound for sensor control device 102. As embodied herein, from Week 1 to Week 13 during the Active Storage mode, the upper and lower error bounds can be limited to less than +/- 5%. If the adaptive adjustment period can be extended to around 14 weeks, it can be reduced by about 1%. From Week 13 to Week 27, the upper and lower error bounds can be limited to less than as much as +/- 15%. Thus, using sensor control device 102 to perform adaptive adjustment and fixed compensation can maintain required sensor sensitivity and extend sensor expiry for about 27 weeks and sensor battery life during its lifespan.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. Thus, the foregoing description of specific embodiments of the disclosed subject matter has been presented for purposes of illustration and description. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It will be apparent to those skilled in the art that various modifications and variations can be made in the method and system of the disclosed subject matter without departing from the scope of the disclosed subject matter. The invention is defined by the appended claims.

**Claims**

1. An analyte monitoring device comprising:

    an analyte sensor,
    a temperature sensor for measuring an external temperature of the analyte sensor,
    one or more processors configured to wake up periodically to generate temperature data indicative of the external temperature of the analyte sensor measured by the temperature sensor, wherein the one or more processors are configured to wake up periodically during storage of the analyte monitoring device prior to activation of the analyte monitoring device;
    a communication module, and
    one or more memories communicatively coupled to the one or more processors, the analyte sensor, the temperature sensor, and the communication module, wherein the one or more processors are configured to:

    generate the temperature data indicative of an external temperature of the analyte sensor measured by the temperature sensor corresponding to a first time;
    calculate a sensitivity adjustment of the analyte sensor based on the temperature data corresponding to the first time;
    add the sensitivity adjustment corresponding to the first time to a total sensitivity adjustment; and
    determine whether the first time is below a predetermined time threshold, and if the first time is below the predetermined time threshold, generate temperature data indicative of an external temperature of the analyte sensor measured by the temperature sensor corresponding to a second time, the second time being after the first time, and calculate a time interval between the second time and the first time.

2. The analyte monitoring device of claim 1, wherein the one or more processors are further configured to:

    determine whether the first time is equal to the predetermined time threshold, and if the first time is equal to the predetermined time threshold, store the total sensitivity adjustment in the one or more memories comprising at least one One Time Programmable (OTP) memory; and/or
    calculate the sensitivity adjustment of the analyte sensor based on the temperature data corresponding to the first time, by multiplying the external temperature corresponding to the first time by a time interval and by a sensor sensitivity change rate.

3. The analyte monitoring device of any preceding claim, wherein the sensor sensitivity change rate is $M = \frac{M_{Tmax}}{T_{max}}$ , wherein $T_{max}$ is a predetermined maximum temperature for which a sensitivity adjustment is calculated, and wherein $M_{Tmax}$ is a sensor sensitivity change rate at the predetermined maximum temperature, optionally wherein $T_{max}$ is 30 °C, wherein $M_{Tmax}$ is 0.003 % /hour, and wherein the time interval between the second time and the first time is 1 hour.

4. The analyte monitoring device of claim 1, wherein the one or more processors are further configured to generate temperature data indicative of the temperature of the analyte sensor measured by the temperature sensor corresponding to a time when the temperature is equal to or below 0 °C, and to calculate a sensitivity adjustment of the analyte sensor based on the temperature data corresponding to the time as 0.

5. The analyte monitoring device of claim 1, wherein the total sensitivity adjustment is a sum of a plurality of sensitivity adjustments corresponding a plurality of times, and wherein the plurality of times includes the first time and the second time.

6. The analyte monitoring device of claim 2, wherein the one or more processors are further configured to determine whether the first time is below the predetermined time threshold, and if the first time exceeds the predetermined time threshold, to calculate a fixed sensitivity adjustment, and to add the fixed sensitivity adjustment to the stored total sensitivity adjustment as a final sensitivity adjustment, optionally wherein the fixed sensitivity adjustment is calculated by multiplying a predetermined maximum temperature for which a sensitivity adjustment is calculated, by a time interval, and by a fixed sensor sensitivity change rate, optionally wherein the fixed sensor sensitivity change rate is a sensor sensitivity change rate at a temperature of half of the predetermined maximum temperature.

7. The analyte monitoring device of claim 6, wherein the one or more processors further are configured to perform a sensitivity adjustment to the analyte sensor, by dividing a sensor sensitivity of the analyte sensor by 100, and by multiplying by a difference between 100 and the final sensitivity adjustment.

8. A computer program product stored on a computer-readable medium comprising instructions to:

   periodically wake up one or more processors of an analyte monitoring device according to claim 1, during storage of the analyte monitoring device prior to activation of the analyte monitoring device, to generate temperature data corresponding to a first time and indicative of an external temperature of an analyte sensor of the analyte monitoring device, measured by a temperature sensor of the analyte monitoring device, the analyte sensor operably connected to the computer-readable medium measured by a temperature sensor operably connected to the computer-readable medium;
   calculate a sensitivity adjustment of the analyte sensor based on the temperature data corresponding to the first time;
   add the sensitivity adjustment corresponding to the first time to a total sensitivity adjustment; and
   determine whether the first time is below a predetermined time threshold, and if the first time is below the predetermined time threshold, generate temperature data indicative of an external temperature of the analyte sensor measured by the temperature sensor corresponding to a second time, the second time being after the first time, and calculate a time interval between the second time and the first time.

9. The computer program product of claim 8 further comprising instructions to:
determine whether the first time is equal to the predetermined time threshold, and if the first time is equal to the predetermined time threshold, store the total sensitivity adjustment in one or more memories comprising at least one One Time Programmable (OTP) memory operably connected to the computer-readable medium, and/or:
calculate the sensitivity adjustment of the analyte sensor based on the temperature data corresponding to the first time, by multiplying the external temperature corresponding to the first time by a time interval and by a sensor sensitivity change rate.

10. The computer program product of claim 8 or 9, wherein the sensor sensitivity change rate is

$$M = \frac{M_{Tmax}}{T_{max}}$$ , wherein $T_{max}$ is a predetermined maximum temperature for which a sensitivity adjustment is calculated, and wherein $M_{Tmax}$ is a sensor sensitivity change rate at the predetermined maximum temperature, optionally wherein $T_{max}$ is 30 °C, wherein $M_{Tmax}$ is 0.003 % /hour, and wherein the time interval between the second time and the first time is 1 hour.

11. The computer program product of claim 8 further comprising instructions to:
generate temperature data indicative of the temperature of the analyte sensor measured by the temperature sensor corresponding to a time when the temperature is equal to or below 0 °C, and to calculate a sensitivity adjustment of the analyte sensor based on the temperature data corresponding to the time as 0.

12. The computer program product of claim 8, wherein the total sensitivity adjustment is a sum of a plurality of sensitivity adjustments corresponding a plurality of times, and wherein the plurality of times includes the first time and the second time.

13. The computer program product of claim 9, further comprising instructions to:
determine whether the first time is below the predetermined time threshold, and if the first time exceeds the predetermined time threshold, to calculate a fixed sensitivity adjustment, and to add the fixed sensitivity adjustment to the stored total sensitivity adjustment as a final sensitivity adjustment, optionally wherein the fixed sensitivity adjustment is calculated by multiplying a predetermined maximum temperature for which a sensitivity adjustment is calculated, by a time interval, and by a fixed sensor sensitivity change rate, optionally wherein the fixed sensor sensitivity change rate is a sensor sensitivity change rate at a temperature of half of the predetermined maximum temperature.

14. The computer program product of claim 13, further comprising instructions to:
perform a sensitivity adjustment to the analyte sensor, by dividing a sensor sensitivity of the analyte sensor by 100, and by multiplying by a difference between 100 and the final sensitivity adjustment.

**Patentansprüche**

1. Analytüberwachungsvorrichtung, umfassend:

einen Analytsensor,
einen Temperatursensor zum Messen einer Außentemperatur des Analytsensors,
einen oder mehrere Prozessoren, die konfiguriert sind, um zum Erzeugen von Temperaturdaten regelmäßig aufzuwachen, welche die Außentemperatur des Analytsensors anzeigen, die von dem Temperatursensor gemessen wird, wobei der eine oder die mehreren Prozessoren konfiguriert sind, um während eines Speicherns der Analytüberwachungsvorrichtung vor einer Aktivierung der Analytüberwachungsvorrichtung regelmäßig aufzuwachen;
ein Kommunikationsmodul, und
einen oder mehrere Speicher, die kommunikativ mit dem einen oder den mehreren Prozessoren, dem Analytsensor, dem Temperatursensor und dem Kommunikationsmodul gekoppelt sind, wobei der eine oder die mehreren Prozessoren konfiguriert sind zum:

Erzeugen der Temperaturdaten, die eine Außentemperatur des Analytsensors anzeigen, die von dem Temperatursensor gemessen wird, und die einem ersten Zeitraum entsprechen;
Berechnen einer Empfindlichkeitsanpassung des Analytsensors basierend auf den Temperaturdaten, die dem ersten Zeitraum entsprechen;
Hinzufügen der Empfindlichkeitsanpassung, die dem ersten Zeitraum entspricht zu einer Gesamtempfindlichkeitsanpassung; und
Ermitteln, ob der erste Zeitraum unter einem vorbestimmten Zeitschwellenwert liegt, und wenn der erste Zeitraum unter dem vorbestimmten Zeitschwellenwert liegt, Erzeugen von Temperaturdaten, die eine Außentemperatur des Analytsensors anzeigen, die von dem Temperatursensor gemessen wird, und die einem zweiten Zeitraum entsprechen, wobei der zweite Zeitraum nach dem ersten Zeitraum liegt, und Berechnen eines Zeitintervalls zwischen dem zweiten Zeitraum und dem ersten Zeitraum.

2. Analytüberwachungsvorrichtung nach Anspruch 1, wobei der eine oder die mehreren Prozessoren außerdem konfiguriert sind zum:

Ermitteln, ob der erste Zeitraum gleich dem vorbestimmten Zeitschwellenwert ist, und wenn der erste Zeitraum gleich dem vorbestimmten Zeitschwellenwert ist, Speichern der Gesamtempfindlichkeitsanpassung in dem einen oder den mehreren Speichern, die mindestens einen einmalprogrammierbaren Speicher (OTP-Speicher) umfassen; und/oder
Berechnen der Empfindlichkeitsanpassung des Analytsensors basierend auf den Temperaturdaten, die dem ersten Zeitraum entsprechen, indem die Außentemperatur, die dem ersten Zeitraum entspricht, mit einem Zeitintervall und mit einer Änderungsrate der Sensorempfindlichkeit multipliziert wird.

3. Analytüberwachungsvorrichtung nach einem der vorhergehenden Ansprüche, wobei die Änderungsrate der Sensorempfindlichkeit gleich $M = \frac{M_{Tmax}}{T_{max}}$ ist, wobei $T_{max}$ eine vorbestimmte Maximaltemperatur ist, für die eine Empfindlichkeitsanpassung berechnet wird, und wobei $M_{Tmax}$ eine Änderungsrate der Sensorempfindlichkeit bei der vorbestimmten Maximaltemperatur ist, wobei $T_{max}$ optional 30 °C beträgt, wobei $M_{Tmax}$ gleich 0,003 %/Stunde ist, und wobei das Zeitintervall zwischen dem zweiten Zeitraum und dem ersten Zeitraum 1 Stunde beträgt.

4. Analytüberwachungsvorrichtung nach Anspruch 1, wobei der eine oder die mehreren Prozessoren außerdem konfiguriert sind zum Erzeugen von Temperaturdaten, welche die Temperatur des Analytsensors anzeigen, die von dem Temperatursensor gemessen wird, die einem Zeitraum entsprechen, wenn die Temperatur gleich 0 °C ist oder darunter liegt, und zum Berechnen einer Empfindlichkeitsanpassung des Analytsensors als 0 basierend auf den Temperaturdaten, die dem ersten Zeitraum entsprechen.

**5.** Analytüberwachungsvorrichtung nach Anspruch 1, wobei die Gesamtempfindlichkeitsanpassung eine Summe von einer Vielzahl von Empfindlichkeitsanpassungen ist, die einer Vielzahl von Zeiträumen entsprechen, und wobei die Vielzahl von Zeiträumen den ersten Zeitraum und den zweiten Zeitraum beinhaltet.

**6.** Analytüberwachungsvorrichtung nach Anspruch 2, wobei der eine oder die mehreren Prozessoren außerdem konfiguriert sind zum Ermitteln, ob der erste Zeitraum unter dem vorbestimmten Zeitschwellenwert liegt, und wenn der erste Zeitraum den vorbestimmten Zeitschwellenwert überschreitet, zum Berechnen einer festen Empfindlichkeitsanpassung und zum Hinzufügen der festen Empfindlichkeitsanpassung zu der gespeicherten Gesamtempfindlichkeitsanpassung als eine endgültige Empfindlichkeitsanpassung, wobei die feste Empfindlichkeitsanpassung optional berechnet wird, indem eine vorbestimmte Maximaltemperatur, für die eine Empfindlichkeitsanpassung berechnet wird, mit einem Zeitintervall und mit einer Änderungsrate der Empfindlichkeitsanpassung multipliziert wird, wobei die feste Empfindlichkeitsanpassung optional eine Änderungsrate der Sensorempfindlichkeit bei einer Temperatur ist, die der Hälfte der Maximaltemperatur entspricht.

**7.** Analytüberwachungsvorrichtung nach Anspruch 6, wobei der eine oder die mehreren Prozessoren außerdem konfiguriert sind zum Durchführen einer Empfindlichkeitsanpassung an dem Analytsensor, indem eine Sensorempfindlichkeit des Analytsensors durch 100 dividiert wird und indem sie mit einer Differenz zwischen 100 und der endgültigen Empfindlichkeitsanpassung multipliziert wird.

**8.** Computerprogrammprodukt, das in einem computerlesbaren Medium gespeichert ist und das Anweisungen umfasst zum:

regelmäßigen Aufwecken von einem oder mehreren Prozessoren einer Analytüberwachungsvorrichtung nach Anspruch 1 während eines Speicherns der Analytüberwachungsvorrichtung vor einer Aktivierung der Analytüberwachungsvorrichtung; Erzeugen von Temperaturdaten, die einem ersten Zeitraum entsprechen und die eine Außentemperatur eines Analytsensors der Analytüberwachungsvorrichtung anzeigen, die von einem Temperatursensor der Analytüberwachungsvorrichtung gemessen wird, wobei der Analytsensor, der funktionsfähig mit dem computerlesbaren Medium verbunden ist, von einem Temperatursensor gemessen wird, der funktionsfähig mit dem computerlesbaren Medium verbunden ist;

Berechnen einer Empfindlichkeitsanpassung des Analytsensors basierend auf den Temperaturdaten, die dem ersten Zeitraum entsprechen; Hinzufügen der Empfindlichkeitsanpassung, die dem ersten Zeitraum entspricht zu einer Gesamtempfindlichkeitsanpassung; und Ermitteln, ob der erste Zeitraum unter einem vorbestimmten Zeitschwellenwert liegt, und wenn der erste Zeitraum unter dem vorbestimmten Zeitschwellenwert liegt, Erzeugen von Temperaturdaten, die eine Außentemperatur des Analytsensors anzeigen, die von dem Temperatursensor gemessen wird, und die einem zweiten Zeitraum entsprechen, wobei der zweite Zeitraum nach dem ersten Zeitraum liegt, und Berechnen eines Zeitintervalls zwischen dem zweiten Zeitraum und dem ersten Zeitraum.

**9.** Computerprogrammprodukt nach Anspruch 8, das außerdem Anweisungen umfasst zum:
Ermitteln, ob der erste Zeitraum gleich dem vorbestimmten Zeitschwellenwert ist, und wenn der erste Zeitraum gleich dem vorbestimmten Zeitschwellenwert ist, Speichern der Gesamtempfindlichkeitsanpassung in dem einen oder den mehreren Speichern, die mindestens einen einmalprogrammierbaren Speicher (OTP-Speicher) umfassen, der funktionsfähig mit dem computerlesbaren Medium verbunden ist, und/oder:
Berechnen der Empfindlichkeitsanpassung des Analytsensors basierend auf den Temperaturdaten, die dem ersten Zeitraum entsprechen, indem die Außentemperatur, die dem ersten Zeitraum entspricht, mit einem Zeitintervall und mit einer Änderungsrate der Sensorempfindlichkeit multipliziert wird.

**10.** Computerprogrammprodukt nach Anspruch 8 oder 9, wobei die Änderungsrate der Sensorempfindlichkeit gleich $M = \frac{M_{Tmax}}{T_{max}}$ ist, wobei $T_{max}$ eine vorbestimmte Maximaltemperatur ist, für die eine Empfindlichkeitsanpassung berechnet wird, und wobei $M_{Tmax}$ eine Änderungsrate der Sensorempfindlichkeit bei der vorbestimmten Maximaltemperatur ist, wobei $T_{max}$ optional 30 °C beträgt, wobei $M_{Tmax}$ gleich 0,003 %/Stunde ist, und wobei das Zeitintervall zwischen dem zweiten Zeitraum und dem ersten Zeitraum 1 Stunde beträgt.

**11.** Computerprogrammprodukt nach Anspruch 8, das außerdem Anweisungen umfasst zum:
Erzeugen von Temperaturdaten, welche die Temperatur des Analytsensors anzeigen, die von dem Temperatursensor gemessen wird, die einem Zeitraum entsprechen, wenn die Temperatur gleich 0 °C ist oder darunter liegt, und zum Berechnen einer Empfindlichkeitsanpassung des Analytsensors als 0 basierend auf den Temperaturdaten, die dem ersten Zeitraum entsprechen.

12. Computerprogrammprodukt nach Anspruch 8, wobei die Gesamtempfindlichkeitsanpassung eine Summe von einer Vielzahl von Empfindlichkeitsanpassungen ist, die einer Vielzahl von Zeiträumen entsprechen, und wobei die Vielzahl von Zeiträumen den ersten Zeitraum und den zweiten Zeitraum beinhaltet.

13. Computerprogrammprodukt nach Anspruch 9, das außerdem Anweisungen umfasst zum:
Ermitteln, ob der erste Zeitraum unter dem vorbestimmten Zeitschwellenwert liegt, und wenn der erste Zeitraum den vorbestimmten Zeitschwellenwert überschreitet, zum Berechnen einer festen Empfindlichkeitsanpassung und zum Hinzufügen der festen Empfindlichkeitsanpassung zu der gespeicherten Gesamtempfindlichkeitsanpassung als eine endgültige Empfindlichkeitsanpassung, wobei die feste Empfindlichkeitsanpassung optional berechnet wird, indem eine vorbestimmte Maximaltemperatur, für die eine Empfindlichkeitsanpassung berechnet wird, mit einem Zeitintervall und mit einer Änderungsrate der Empfindlichkeitsanpassung multipliziert wird, wobei die feste Empfindlichkeitsanpassung optional eine Änderungsrate der Sensorempfindlichkeit bei einer Temperatur ist, die der Hälfte der Maximaltemperatur entspricht.

14. Computerprogrammprodukt nach Anspruch 13, das außerdem Anweisungen umfasst zum:
Durchführen einer Empfindlichkeitsanpassung an dem Analytsensor, indem eine Sensorempfindlichkeit des Analytsensors durch 100 dividiert wird und indem sie mit einer Differenz zwischen 100 und der endgültigen Empfindlichkeitsanpassung multipliziert wird.

## Revendications

1. Dispositif de surveillance d'analyte comprenant :

un capteur d'analyte,
un capteur de température pour mesurer une température externe du capteur d'analyte,
un ou plusieurs processeurs configurés pour se réveiller périodiquement afin de générer des données de température indiquant la température externe du capteur d'analyte mesurée par le capteur de température, le ou les processeurs étant configurés pour se réveiller périodiquement pendant le stockage du dispositif de surveillance d'analyte avant l'activation du dispositif de surveillance d'analyte ;
un module de communication, et

une ou plusieurs mémoires couplées de manière communicative au ou aux processeurs, au capteur d'analyte, au capteur de température et au module de communication, le ou les processeurs étant configurés pour :

générer les données de température indiquant une température externe du capteur d'analyte mesurée par le capteur de température correspondant à un premier moment ;
calculer un ajustement de sensibilité du capteur d'analyte sur la base des données de température correspondant au premier moment ;
ajouter l'ajustement de sensibilité correspondant au premier moment à un ajustement de sensibilité total ; et
déterminer si le premier moment est inférieur à un seuil de temps prédéterminé, et si le premier moment est inférieur au seuil de temps prédéterminé, générer des données de température indiquant une température externe du capteur d'analyte mesurée par le capteur de température correspondant à un deuxième moment, le deuxième moment étant postérieur au premier moment, et calculer un intervalle de temps entre le deuxième moment et le premier moment.

2. Dispositif de surveillance d'analyte selon la revendication 1, le ou les processeurs étant en outre configurés pour :

déterminer si le premier moment est égal au seuil de temps prédéterminé, et si le premier moment est égal au seuil de temps prédéterminé, stocker l'ajustement de sensibilité total dans la ou les mémoires comprenant au moins une mémoire programmable une seule fois (OTP) ; et/ou
calculer l'ajustement de sensibilité du capteur d'analyte sur la base des données de température correspondant au premier moment, en multipliant la température externe correspondant au premier moment par un intervalle de temps et par un taux de variation de sensibilité de capteur.

3. Dispositif de surveillance d'analyte selon l'une quelconque des revendications précédentes, le taux de variation de sensibilité de capteur étant $M = \frac{M_{Tmax}}{Tmax}$, $T_{max}$ étant une température maximale prédéterminée pour laquelle un ajustement de sensibilité est calculé, et $M_{Tmax}$ étant un taux de variation de sensibilité de capteur à la température maximale prédéterminée, éventuellement $T_{max}$

étant égal à 30 °C, $M_{Tmax}$ étant égal à 0,003 %/heure, et l'intervalle de temps entre le deuxième moment et le premier moment étant de 1 heure

4. Dispositif de surveillance d'analyte selon la revendication 1, le ou les processeurs étant en outre configurés pour générer des données de température indiquant la température du capteur d'analyte mesurée par le capteur de température correspondant à un moment où la température est égale ou inférieure à 0 °C, et pour calculer un ajustement de sensibilité du capteur d'analyte sur la base des données de température correspondant à ce moment, en le fixant à 0.

5. Dispositif de surveillance d'analyte selon la revendication 1, l'ajustement de sensibilité total étant une somme d'une pluralité d'ajustements de sensibilité correspondant à une pluralité de moments, et ladite pluralité de moments comprenant le premier moment et le deuxième moment.

6. Dispositif de surveillance d'analyte selon la revendication 2, le ou les processeurs étant en outre configurés pour déterminer si le premier moment est inférieur au seuil de temps prédéterminé, et si le premier moment dépasse le seuil de temps prédéterminé, pour calculer un ajustement de sensibilité fixe, et pour ajouter l'ajustement de sensibilité fixe à l'ajustement de sensibilité total stocké en tant qu'ajustement de sensibilité final, éventuellement, l'ajustement de sensibilité fixe étant calculé en multipliant une température maximale prédéterminée pour laquelle un ajustement de sensibilité est calculé, par un intervalle de temps, et par un taux de variation de sensibilité de capteur fixe, éventuellement, le taux de variation de sensibilité de capteur fixe étant un taux de variation de sensibilité de capteur à une température égale à la moitié de la température maximale prédéterminée.

7. Dispositif de surveillance d'analyte selon la revendication 6, le ou les processeurs étant en outre configurés pour effectuer un ajustement de sensibilité du capteur d'analyte, en divisant une sensibilité de capteur du capteur d'analyte par 100, puis en la multipliant par une différence entre 100 et l'ajustement de sensibilité final.

8. Produit de programme informatique stocké sur un support lisible par ordinateur, comprenant des instructions pour :

réveiller périodiquement un ou plusieurs processeurs d'un dispositif de surveillance d'analyte selon la revendication 1, pendant le stockage du dispositif de surveillance d'analyte avant l'activation du dispositif de surveillance d'analyte, afin de générer des données de température correspondant à un premier moment et indiquant une température externe d'un capteur d'analyte du dispositif de surveillance d'analyte mesurée par un capteur de température du dispositif de surveillance d'analyte, le capteur d'analyte étant connecté de manière opérationnelle au support lisible par ordinateur mesuré par un capteur de température connecté de manière opérationnelle au support lisible par ordinateur ;
calculer un ajustement de sensibilité du capteur d'analyte sur la base des données de température correspondant au premier moment ;
ajouter l'ajustement de sensibilité correspondant au premier moment à un ajustement de sensibilité total ; et
déterminer si le premier moment est inférieur à un seuil de temps prédéterminé, et si le premier moment est inférieur au seuil de temps prédéterminé, générer des données de température indiquant une température externe du capteur d'analyte mesurée par le capteur de température correspondant à un deuxième moment, le deuxième moment étant postérieur au premier moment, et calculer un intervalle de temps entre le deuxième moment et le premier moment.

9. Produit de programme informatique selon la revendication 8, comprenant en outre des instructions pour :
déterminer si le premier moment est égal au seuil de temps prédéterminé, et si le premier moment est égal au seuil de temps prédéterminé, stocker l'ajustement de sensibilité total dans une ou plusieurs mémoires comprenant au moins une mémoire programmable une seule fois (OTP) connectée de manière opérationnelle au support lisible par ordinateur, et/ou :
calculer l'ajustement de sensibilité du capteur d'analyte sur la base des données de température correspondant au premier moment, en multipliant la température externe correspondant au premier moment par un intervalle de temps et par un taux de variation de sensibilité de capteur.

10. Produit de programme informatique selon la revendication 8 ou 9, le taux de variation de sensibilité de capteur étant $M = \dfrac{M_{Tmax}}{Tmax}$, $T_{max}$ étant une température maximale prédéterminée pour laquelle un ajustement de sensibilité est calculé, et $M_{Tmax}$ étant un taux de variation de sensibilité de capteur à la température maximale prédéterminée, éventuellement $T_{max}$ étant de 30 °C, $M_{Tmax}$ étant de 0,003 %/heure, et l'intervalle de temps entre le deuxième moment et le premier moment étant d'une heure.

**11.** Produit de programme informatique selon la revendication 8, comprenant en outre des instructions pour :

générer des données de température indiquant la température du capteur d'analyte mesurée par le capteur de température correspondant à un moment où la température est égale ou inférieure à 0 °C, et pour calculer un ajustement de sensibilité du capteur d'analyte sur la base des données de température correspondant à ce moment, en le fixant à 0.

**12.** Produit de programme informatique selon la revendication 8, l'ajustement de sensibilité total étant une somme d'une pluralité d'ajustements de sensibilité correspondant à une pluralité de moments, et la pluralité de moments comprenant le premier moment et le deuxième moment.

**13.** Produit de programme informatique selon la revendication 9, comprenant en outre des instructions pour :

déterminer si le premier moment est inférieur au seuil de temps prédéterminé, et si le premier moment dépasse le seuil de temps prédéterminé, calculer un ajustement de sensibilité fixe, et ajouter l'ajustement de sensibilité fixe à l'ajustement de sensibilité total stocké en tant qu'ajustement de sensibilité final, éventuellement, l'ajustement de sensibilité fixe étant calculé en multipliant une température maximale prédéterminée pour laquelle un ajustement de sensibilité est calculé, par un intervalle de temps, et par un taux de variation de sensibilité de capteur fixe, éventuellement, le taux de variation de sensibilité de capteur fixe étant un taux de variation de sensibilité de capteur à une température égale à la moitié de la température maximale prédéterminée.

**14.** Produit de programme informatique selon la revendication 13, comprenant en outre des instructions pour :

effectuer un ajustement de sensibilité de capteur du capteur d'analyte, en divisant une sensibilité du capteur d'analyte par 100, et en la multipliant par une différence entre 100 et l'ajustement de sensibilité final.

**FIG. 1 A**

EP 4 408 284 B1

FIG. 1B

EP 4 408 284 B1

| Display 122 | Input Component 121 |

**Reader Device 120**

Communications Processor 222

Memory 223

Applications Processor 224

Memory 225

206

229

RF Transceiver 228

Memory 230

Multi-Functional (WiFi) (NFC) (Bluetooth, BTLE) (GPS) 232

234

Power Supply 226

Power Management 238

**FIG. 2A**

FIG. 2B

**FIG. 2C**

Sensor Electronics 160

ASIC 161 · AFE 162 · Power Mgmt. Circuitry 164 · Communication Circuitry 168 · Processor 166 · Memory 163 · Power Source 172 · Analyte Sensor 104 · 171 · Sensor Control Device 102

**FIG. 2D**

Sensor Electronics 160

Chip 174 · Power Mgmt. Circuitry 164 · Memory 165 · Processor 166 · Communication Circuitry 168 · Power Source 170 · AFE 162 · Memory 163 · ASIC 161 · Analyte Sensor 104 · 171 · Sensor Control Device 102

**102**
**Sensor Control Device**

**5060**
**Sensing Hardware**

**5050**
**Power Module**

**5040**
**Communication Module**

**5000**
**ASIC**

**5010**
**Microcontroller**

**5030**
**Storage**

**5020**
**Memory**

**5025**
**NFC**

**5041**
**BLE**

**5043**
**Memory**

*FIG. 2E*

EP 4 408 284 B1

**FIG. 3A**

**FIG. 3B**

**FIG. 3C**

**FIG. 3D**

**FIG. 3E**

**FIG. 3F**

**FIG. 4A**

**FIG. 4B**

**FIG. 4C**

FIG. 4D

FIG. 4E

EP 4 408 284 B1

FIG. 4F

FIG. 4G

FIG. 6A

FIG. 5

FIG. 6B

FIG. 7A

**FIG. 7B**

**FIG. 8A**

**FIG. 8B**

**FIG. 8C**

**FIG. 9A**

**FIG. 9B**

**FIG. 10A**

**FIG. 10B**

FIG. 11A

FIG. 11B

**FIG. 11C**

FIG. 12B

FIG. 12A

**FIG. 12C**

FIG. 13A

FIG. 13B

**FIG. 13C**

FIG. 13D

FIG. 13E

FIG. 13F

FIG. 14

6000

FIG. 15

FIG. 16

EP 4 408 284 B1

600

| 102 | 120 |
|-----|-----|
| Sensor Control Device | Data Receiving Device |

605
Sensor Activation Command

610
Collect data

615
Authentication Request

620
Mutual Authentication

625
Encrypted Sensor Secret

630
Determine sensor-
unique encryption key

635
Encrypt payload using
sensor-unique encryption
key

640
Encrypted Payload

645
Decrypt payload using
sensor-unique encryption
key

*FIG. 17*

Adjusted 2 and 28 Degrees Data

FIG. 18

○ = 2, ● = 28

1801

1805

Time (Weeks)

Adjusted %Difference from Day 0

1900

```
┌─────────────────────────────┐
│           1901              │
│   Receive configure sensor  │
│   control device command    │
└─────────────────────────────┘
              │
              ▼
┌─────────────────────────────┐
│           1902              │
│   Go to Active Storage Mode │
└─────────────────────────────┘
              │
              ▼
┌─────────────────────────────┐
│           1903              │
│   Measure temperature       │
└─────────────────────────────┘
```

**1910**
Wait for predetermined time interval

**1903** Measure temperature

**1905** Calculate adjustment to sensor sensitivity

**1907** Add to previous sensor sensitivity adjustments

**1909** Time greater than threshold?

No

Yes

**1911** Store total sensitivity adjustment in memory

**1913** Go to Normal Storage Mode

**1915** Sensor control device activated

**1917** Go to Active Mode

**1919** Calculate final sensitivity adjustment from fixed compensation and stored total sensitivity adjustment

**1921** Adjust sensor sensitivity

**1923** Continue with Active Mode

*FIG. 19*

FIG. 20

FIG. 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9977010 B
- US 10908114 B
- US 20130150691 A
- US 20210204841 A
- WO 2018136898 A, Rao
- WO 2019236850 A, Thomas
- WO 2019236859 A, Thomas
- WO 2019236876 A, Thomas
- US 20200196919 A
- US 20160331283 A
- US 2018023552 A
- US 20140171771 A
- US 20100230285 A
- US 20190274598 A